# EUROPÄISCHE PATENTSCHRIFT

(11) **EP 4 337 311 B1**
(45) Veröffentlichungstag und Bekanntmachung des Hinweises auf die Patenterteilung: **25.02.2026**
(21) Anmeldenummer: 22724419.1
(22) Anmeldetag: 10.05.2022
(51) Int. Cl.: A61N 5/06, A61B 1/00, A61B 1/06, A61B 17/34

(54) **LICHTAPPLIKATORSYSTEM MIT VORSTICHHILFE**
LIGHT APPLICATOR SYSTEM WITH PRE-PIERCING AID
SYSTÈME APPLICATEUR DE LUMIÈRE AVEC UN ACCESSOIRE DE PRÉ-PERÇAGE

(30) Priorität: 14.05.2021 DE 102021204909
(43) Veröffentlichungstag der Anmeldung: 20.03.2024
(73) Patentinhaber: Richard Wolf GmbH, 75438 Knittlingen (DE)
(72) Erfinder: WEBER, Bernd Claus, 76228 Karlsruhe (DE); SIEBER, Stephan, 75438 Knittlingen-Freudenstein (DE)
(74) Vertreter: Patentanwälte Hemmer Lindfeld Frese Partnerschaft mbB
(86) Internationale Anmeldenummer: PCT/DE2022/200098
(87) Internationale Veröffentlichungsnummer: WO 2022/237944

(56) Entgegenhaltungen:
- DE-A1- 10 300 787
- DE-A1- 102019 129 556
- GB-A- 2 514 444
- US-A- 6 048 359
- US-A1- 2008 249 517
- US-A1- 2012 041 521
- US-A1- 2019 002 594

## Beschreibung

Die vorliegende Offenbarung betrifft ein Lichtapplikatorsystem zur Untersuchung und/oder Behandlung eines organischen Körpers, insbesondere zur photodynamischen Therapie (PDT) von pathologischem Gewebe.

Es ist bekannt, Endoskope dazu zu nutzen, um Videoaufnahmen vom Inneren eines menschlichen oder tierischen Körpers zu Zwecken der medizinischen Diagnose und/oder Therapie zu machen. Dabei ist es ein ständiges Bestreben, den Einführabschnitt von Endoskopen möglichst dünn auszugestalten, damit möglichst kleine Hohlräume eingesehen werden können und das Gewebe dabei möglichst wenig in Mitleidenschaft zu ziehen.

Endoskope werden allerdings nicht nur dazu genutzt, um Bildoder Videoaufnahmen zu machen, sondern auch als Diagnose- oder Therapiemittel selbst eingesetzt. Beispielsweise für die Detektion und Lokalisierung von prä- und frühmalignem Gewebe kann eine Fluoreszenz-Endoskopie eingesetzt werden, bei der es nicht auf eine natürliche Echtfarb-Darstellung des Gewebes ankommt, sondern lediglich auf eine Fluoreszenzanregung, mit der sich pathologisches Gewebe von gesundem Gewebe unterscheiden lässt. Dabei kann das mittels Lichtstrahlung angeregte pathologische Gewebe selbst oder eine auf pathologisches Gewebe hinweisende Bakterienansammlung spezifisch fluoreszieren und so gegenüber dem umliegenden gesunden Gewebe erkennbar lokalisiert werden. Die Fluoreszenz-Endoskopie kann beispielsweise im Rahmen einer photodynamischen Diagnose (PDD) und/oder photodynamischen Therapie (PDT) mittels eines Photosensibilisators bzw. Markerstoffs durchgeführt werden, der sich selektiv an pathologischem Gewebe anreichert.

Bei der photodynamischen Therapie (PDT) wird Licht mittels eines Lichtapplikators unmittelbar auf oder sogar in pathologisches Gewebe appliziert, um lichtinduziert die Bildung von Sauerstoffradikalen mittels des örtlich begrenzt angereicherten Photosensibilisators bzw. Markerstoffs zu fördern und dadurch das pathologische Gewebe, wie etwa einen Tumor, zu zerstören. Typischerweise wird dazu Laserlicht in einen Lichtleiter eingekoppelt und zum Gewebe geleitet. Wenn das pathologische Gewebe flächig auf einer äußeren Oberfläche, z. B. der Haut, oder einer inneren Oberfläche, z. B. der Speiseröhreninnenfläche oder Darmwandung, angeordnet ist, dann kann das Therapielicht relativ einfach ausgekoppelt und auf die pathologische Gewebefläche gestrahlt werden. Erstreckt sich allerdings das pathologische Gewebe über ein Volumen, so kann wegen der begrenzten Eindringtiefe des Lichts in das Gewebe nicht immer ein Tumor von "außen" effektiv bestrahlt werden. In diesem Fall ist die PDT besonders effektiv, wenn das Licht vom Inneren des pathologischen Gewebevolumens aus möglichst isotrop abgestrahlt wird. Dazu muss der Lichtapplikator in das pathologische Gewebe eingestochen werden. Dies wird auch als interstitielle (durch innere Oberflächen) und/oder perkutane (durch die Haut) PDT bezeichnet.

In der US 6,048,359 ist beispielsweise beschrieben, wie mehrere Lichtapplikatoren mit Hilfe eines Positionierungsrasters in pathologisches Gewebe eingestochen werden.

Es hat sich bei den bekannten Lösungen allerdings als eine technische Herausforderung herausgestellt, dass das Durchstechen der Haut mit einem sehr dünnen und langen Lichtapplikator in manchen Fällen eine so hohe manuelle Kraft erfordert, dass sich der Lichtapplikator dabei verbiegt und das tieferliegende Zielgewebe deshalb verfehlt wird. Dieses Problem besteht insbesondere dann, wenn das Zielgewebe relativ weit im Körperinneren liegt, also der Lichtapplikator relativ lang sein muss, aber möglichst dünn ausgestaltet ist, um den Einstich möglichst minimalinvasiv auszugestalten.

Daraus ergibt sich die Aufgabe, ein Lichtapplikatorsystem bereitzustellen, mit dem tief liegendes Gewebe in einem organischen Körper möglichst zielgenau und minimalinvasiv zur Untersuchung und/oder Behandlung erreicht werden kann.

Gemäß der vorliegenden Offenbarung wird zur Lösung dieses Problems ein Lichtapplikatorsystem nach Anspruch 1 zur Untersuchung und/oder Behandlung von einem organischen Körper bereitgestellt. Dies gewährleistet, dass die Nadelspitze des Lichtapplikators die von der Nadelspitze der Vorstichhilfe in der Haut des Patienten vorab erzeugte kleine Öffnung von selbst oder "automatisch" findet, indem der Lichtapplikator lediglich in die mindestens eine Aufnahme des Positionierungselements eingeführt und innerhalb dieser Aufnahme in distale Richtung vorwärts bewegt wird. Die Bedienperson muss also die kleine vorgestochene Hautöffnung nicht suchen und benötigt kein manuelles Geschick, um diese zu treffen. Durch den Vorstich wird der von der zähen Haut verursachte Widerstand, den der Applikator beim Einführen bzw. Vorschieben in bzw. durch die Haut erfährt, reduziert und dadurch die Gefahr eines Verbiegens des Lichtapplikators bzw. dessen Einführabschnitts verringert, damit die Nadelspitze des Lichtapplikators den Zielpunkt im organischen Körper erreicht.

Bei dem hier offenbarten Lichtapplikator wird vorzugsweise kein Laserlichtleiter eingesetzt, sondern das Diagnose- oder Therapielicht in situ am distalen Ende des Lichtapplikators durch ein aktiv lichtemittierendes Element, z.B. eine miniaturisierte LED erzeugt, beispielsweise mit einer lateralen Breite von weniger als 1 mm. Als ein "aktiv" lichtemittierendes Element soll hier nur ein lichtemittierendes Element verstanden werden, das elektrische Energie aufnimmt und in Licht umwandelt, d.h. kein Lichtleiter, der lediglich Licht weiterleitet und/oder auskoppelt. Ein teurer Laser wird bei einem aktiv lichtemittierenden Element nicht benötigt, sodass die Kosten stark reduziert sind. Der hierin offenbarte Lichtapplikator kann sehr günstig hergestellt werden und somit als steriler Einweg-Artikel zum einmaligen Gebrauch realisiert werden, was aufwendige Reinigungen und Sterilisationen beim Anwender obsolet macht. Bei größeren Tumoren oder ganzen pathologischen Organen oder Organbereichen kann das Lichtapplikatorsystem eine Mehrzahl an Lichtapplikatoren aufweisen, die gleichzeitig für die PDT eingesetzt werden, indem sie verteilt über das gesamte Organ eingestochen werden, um das gesamte Organ homogen auszuleuchten. Da sich der Photosensibilisator bzw. Markerstoff selektiv nur in pathologischem Gewebe anreichert und dort unter dem Lichteinfluss reagiert und die applizierten Lichtmengen vergleichsweise gering sind, wird gesundes Gewebe durch das Licht nicht beschädigt. Zum einen muss das pathologische Gewebe dann zuvor nicht mehr so genau lokalisiert werden und zum anderen wird das Risiko verringert, dass pathologisches Gewebe unbemerkt untherapiert bleibt.

Vorzugsweise weist der Einführabschnitt des Lichtapplikators eine sich zumindest teilweise distal von der LED angeordnete und sich distalwärts spitz verjüngende Nadelspitze mit einem lichttransparenten Streukörper zur Streuung des Lichts der LED auf. Die Nadelspitze des Einführabschnitts des Lichtapplikators dient einerseits dazu, den Einstich möglichst minimalinvasiv zu gestalten, und andererseits dazu, das Licht der LED in einem möglichst großen Raumwinkel, z.B. über 3π, möglichst isotrop und/oder homogen zu streuen.

Für die perkutane PDT mit mehreren Lichtapplikatoren kann es sinnvoll sein, dass das Positionierungselement eine Vielzahl von Aufnahmen aufweist und eine unweit oder direkt an oder auf die Haut des Patienten definiert platzierbare und/oder klebbare organspezifische Schablone bildet, um einem Anwender damit Einstichstellen, -winkel und/oder -tiefen für die Lichtapplikatoren anzuzeigen und eine möglichst vollständige und weitgehend homogene Ausleuchtung des Organs zu erzielen.

Der Lichtapplikator kann allerdings nicht nur für die Therapie eingesetzt werden, sondern auch für die Untersuchung, also die Diagnose. Insbesondere im Zusammenspiel mit einem Endoskop oder mit dem Lichtapplikator als Teil eines Endoskops kann die durch den Lichtapplikator erzeugte Fluoreszenz eines in pathologischem Gewebe angereicherten Photosensibilisators bzw. Markerstoffs beobachtet werden.

Durch die Vorstichhilfe kann die Haut vor dem eigentlichen Einstich des Lichtapplikators vorgestochen werden, wodurch der Widerstand, den die Haut beim eigentlichen Einstich des Lichtapplikators verursacht, erheblich reduziert ist. Vorzugsweise wird die Haut durch die Vorstichhilfe nicht nur vorgestochen, sondern auch durch den Vorstich aufgeweitet und/oder eingeschnitten, wodurch nicht nur der anfängliche Widerstand gegen den Einstich des Lichtapplikators, sondern auch der nachfolgende Reibwiderstand am Lichtapplikator beim Durchstich durch die Haut reduziert ist. Vorzugsweise ist die Vorstichhilfe bzw. deren Nadelspitze aber dennoch so gestaltet, dass der Vorstich möglichst atraumatisch und minimalinvasiv geschieht, sodass die Öffnung in der Haut nach dem Zurückziehen der Vorstichhilfe aus der Haut nur sehr klein ist, was für das anschließende Auffinden und zielgenaue Treffen dieser Öffnung mit dem Lichtapplikator bzw. dessen Nadelspitze zwar einerseits eine große Herausforderung darstellt, andererseits aber oft ausreicht, um den von der Haut erzeugten Widerstand beim Einführen des Lichtapplikators zu reduzieren.

Erfindungsgemäß weist das Lichtapplikatorsystem ferner einen Anschlag auf, der eine maximale Vorstichtiefe der Vorstichhilfe definiert. Dies hat den Vorteil, dass die Vorstichhilfe nicht versehentlich zu tief eingestochen wird.

In einer hier nicht beanspruchten Ausführungsform des Lichtapplikatorsystems kann die Vorstichhilfe vorzugsweise eine vom Lichtapplikator separate Komponente sein, welche nach einem Vorstich in den organischen Körper aus der zumindest einen Aufnahme des Positionierungselements entnehmbar ist, um anschließend den Lichtapplikator zur Untersuchung und/oder Behandlung des organischen Körpers durch die zumindest eine Aufnahme des Positionierungselements in den vorgestochenen organischen Körper einzuführen.

Die Vorstichhilfe kann dabei biegesteifer ausgestaltet werden als der Lichtapplikator, da die Vorstichhilfe kürzer und/oder dicker als der Lichtapplikator ausgestaltet werden kann.

Optional kann die Vorstichhilfe einen Vorstichhilfeschaft mit einer axialen Länge und einem Durchmesser aufweisen, die ein erstes Länge-Durchmesser-Verhältnis bestimmen, und der Einführabschnitt des Lichtapplikators kann eine axiale Länge und einen Durchmesser aufweisen, die ein zweites Länge-Durchmesser-Verhältnis bestimmen, wobei das zweite Länge-Durchmesser-Verhältnis wesentlich größer ist als das erste Länge-Durchmesser-Verhältnis. Dadurch kann die Vorstichhilfe wesentlich biegesteifer ausgestaltet werden als der Einführabschnitt des Lichtapplikators. Zusätzlich kann die Biegesteifigkeit der Vorstichhilfe im Hinblick auf die Materialwahl optimiert sein, z.B. durch einen massiven, harten Stahlkern, was beim Lichtapplikator aufgrund zusätzlicher, anderer Anforderungen (beispielsweise weicher Kupferkern für eine gute Wärmeleitung vom distalen aktiv lichtemittierenden Element nach proximal) so ggf. nicht immer möglich ist.

Optional kann die Nadelspitze der Vorstichhilfe zumindest eine oder mehrere Schneidkanten aufweisen, wodurch die vorgestochene Haut eingeschnitten wird, was die Hautspannung und damit den durch die Haut bewirkten Widerstand beim Durchstechen des Einführabschnitts des Lichtapplikators reduziert.

Optional kann zumindest eine dieser Schneidkanten als distal vorstehende Schneidklinge ausgebildet sein, was das Einschneiden der Haut vereinfachen kann und/oder einen noch saubereren Schnitt erzeugen kann.

Unabhängig von der Ausführung im Hinblick auf optionale Schneidkanten oder Schneidklingen ist die Nadelspitze der Vorstichhilfe bzw. deren Spitze in Bezug auf den Vorstichhilfeschaft aber immer axial zentrisch angeordnet. Ebenso ist auch die Nadelspitze des Lichtapplikators bzw. deren Spitze in Bezug auf den Einführabschnitt des Lichtapplikators immer axial zentrisch angeordnet.

Optional kann die Vorstichhilfe einen Vorstichhilfeschaft mit einem Durchmesser aufweisen, der identisch ist mit dem Durchmesser des Einführabschnitts des Lichtapplikators, und die beide passgenau in einen Innendurchmesser der mindestens einen Aufnahme des Positionierungselements passen. Die gegenüber dem Einführabschnitt des Lichtapplikators geforderte größere Biegesteifigkeit des Vorstichhilfeschafts wird dadurch erreicht, dass der Vorstichhilfeschaft kürzer ausgeführt wird. Vorzugsweise ist Nadelspitze der Vorstichhilfe mit zumindest einer Schneidkante oder Schneidklinge versehen. Durch die mindestens eine Kante oder Klinge kann die Haut beim Vorstich eingeschnitten werden, damit diese beim Durchstechen des Lichtapplikators weniger Widerstand bietet.

Optional kann die Vorstichhilfe einen Vorstichhilfeschaft mit einem Durchmesser aufweisen, der passgenau in einen Innendurchmesser der mindestens einen Aufnahme des Positionierungselements passt und größer als ein Durchmesser des Einführabschnitts des Lichtapplikators ist, wobei das Lichtapplikatorsystem ferner eine Adapterhülse aufweist, deren Innendurchmesser passgenau dem Durchmesser des Einführabschnitts des Lichtapplikators entspricht und die zumindest abschnittsweise einen Außendurchmesser aufweist, der passgenau in einen Innendurchmesser der mindestens einen Aufnahme des Positionierungselements passt. Durch den dickeren Vorstichhilfeschaft kann die Haut beim Vorstich gedehnt werden, damit diese beim Durchstechen des Lichtapplikators weniger Widerstand bietet. Optional kann dabei die Adapterhülse als Schutzhülse einer Nadelspitze des Einführabschnitts des Lichtapplikators fungieren und mit dem Einführabschnitt axial beweglich und unverlierbar gekoppelt sein.

Optional kann die Vorstichhilfe so ausgeführt sein, dass sie in Verbindung mit einer Adapterhülse, die als Schutzhülse für die Nadelspitze der Vorstichhilfe fungiert, in die Aufnahme des Positionierungselements eingeführt wird, wobei der Innendurchmesser der Adapterhülse passgenau dem Durchmesser des Einführabschnitts der Vorstichhilfe entspricht und die zumindest abschnittsweise einen Außendurchmesser aufweist, der passgenau in einen Innendurchmesser der mindestens einen Aufnahme des Positionierungselements passt. Ist dann die Nadelspitze der Vorstichhilfe beispielsweise mit scharfen Schneidkanten oder gar mit einer Schneidklinge ausgebildet, kann die Haut beim Vorstich eingeschnitten werden, damit diese beim Durchstechen des Lichtapplikators weniger Widerstand bietet.

Optional können sowohl der Lichtapplikator als auch die Vorstichhilfe so ausgeführt sein, dass sie in Verbindung mit einer Adapterhülse, die als Schutzhülse für die jeweilige Nadelspitze fungiert, in die Aufnahme des Positionierungselements eingeführt werden, wobei die Adapterhülse des Lichtapplikators mit der Adapterhülse der Vorstichhilfe zumindest in Bezug auf Außen- und Innendurchmesser, ggf. aber sogar vollkommen identisch sind.

Erfindungsgemäß ist die Vorstichhilfe in den Lichtapplikator integriert, wobei die Nadelspitze der Vorstichhilfe eine sich zumindest teilweise distal von der LED angeordnete und sich distalwärts spitz verjüngende Nadelspitze des Lichtapplikators mit einem lichttransparenten Streukörper zur Streuung des Lichts der LED ist, wobei der Anschlag derart positionierbar und/oder formbar ist, dass er in einer Anschlagsposition eine maximale Vorstichtiefe des Lichtapplikators definiert und in einer Nichtanschlagsposition eine größere Einstichtiefe des Lichtapplikators erlaubt. Die Nadelspitze des Lichtapplikators bildet also hier die Nadelspitze der Vorstichhilfe und der Einführabschnitt des Lichtapplikators bildet den Vorstichhilfeschaft. Vorzugsweise wird der Anschlag der Vorstichhilfe durch eine axial auf dem Einführabschnitt des Lichtapplikators positionier- und fixierbare Stabilisationshülse gebildet.

Die Vorstichhilfe kann optional eine Stabilisationshülse aufweisen, die vorzugsweise den Anschlag bildet und axial auf einem Vorstichhilfeschaft der Vorstichhilfe beweglich und in einer wählbaren Axialposition auf dem Vorstichhilfeschaft fixierbar ist. Dadurch ist die Vorstichtiefe an den Anwendungsfall anpassbar. Die Stabilisationshülse kann zusätzlich als Adapterhülse und/oder Schutzhülse einer Nadelspitze des Einführabschnitts des Lichtapplikators fungieren oder als separate Komponente ausgebildet sein. Die Stabilisationshülse kann dabei den dünnen Einführabschnitt des Lichtapplikators proximal des Positionierungselements stabilisieren und damit für den Vorstich durch die Haut biegesteifer machen.

Optional kann die Vorstichhilfe und/oder der Lichtapplikator proximalseitig ein Griffelement zur manuellen Positionierung und Ausrichtung aufweisen. Vorzugsweise gibt es nur ein Griffelement. Allerdings kann es bei der Ausführungsform mit separater Vorstichhilfe auch nur ein Griffelement geben, wenn dieses umsetzbar sowohl an ein proximales Ende des Einführabschnitt als auch ein proximales Ende der Vorstichhilfe passend ansetzbar ist. Die Vorstichhilfe und der Lichtapplikator können alternativ jeweils ein eigenes Griffelement aufweisen. Optional kann die Axialposition der Vorstichhilfe und/oder des Einführabschnitts relativ zum Positionierungselement manuell durch Positionieren des Griffelements in Axialrichtung einstellbar sein.

Optional kann das Griffelement den Anschlag bilden und axial auf der Vorstichhilfe und/oder dem Einführabschnitt beweglich und in einer wählbaren Axialposition fixierbar sein. Dann kann das Griffelement für den Vorstich durch die Haut so weit distalwärts geschoben werden bis es an das Positionierungselement stößt. Die Vorstichhilfe kann dann sehr kurz und entsprechend biegesteif ausgestaltet sein.

Optional kann das Lichtapplikatorsystem eine Widerstandskomponente aufweisen, welche eine proximalwärtige Widerstandskraft gegen eine distalwärtig manuell auf die Vorstichhilfe ausgeübte Kraft bis zu einer maximalen Widerstandskraft ausübt und die Vorstichhilfe schlagartig distalwärts freigibt, sobald die distalwärtige manuelle Kraft auf die Vorstichhilfe die maximale Widerstandskraft überschreitet. Dies ist besonders vorteilhaft, wenn die Haut relativ weit distalwärts nachgibt und die von der Haut aufgebrachte proximalwärts gerichtete Reaktionskraft relativ lange so klein bleibt, dass Haut nur eingedellt wird, aber der Druck der Nadelspitze der Vorstichhilfe für einen Einstich in die Haut nicht ausreicht. In diesem Fall kann sich, sofern die Vorstichhilfe noch nicht angeschlagen ist, die Haut über eine relativ große distalwärtige Strecke mit sich langsam vergrößernder Reaktionskraft dehnen und dann, wenn der Druck der Nadelspitze der Vorstichhilfe für einen Vorstich durch die Haut erreicht ist, schlagartig proximalwärts zurückschnellen. Die Nadelspitze der Vorstichhilfe könnte dann schon zu weit durch die Haut in darunter liegendes Gewebe gestochen sein, was vermieden werden soll, um den Vorstich möglichst minimalinvasiv zu gestalten. Dieses Problem wird dadurch erschwert, dass das Positionierungselement, beispielsweis in Form einer auf die Haut geklebten Einstichschablone, die Sicht der Bedienperson auf die Nadelspitze und die Haut versperren kann.

Zur Lösung dieses Problems übernimmt die Widerstandskomponente von der Haut die Funktion des Aufbauens einer Reaktionskraft, um den für den Vorstich notwendigen Druck auf die Haut aufzubauen. Sobald die Bedienperson die manuelle distalwärtige Kraft derart erhöht hat, dass die maximale Widerstandskraft überschritten ist, erfährt die Vorstichhilfe schlagartig einen distalwärtigen Kraftstoß mit entsprechender Beschleunigung, um den für den Vorstich notwendigen Druck auf die Haut aufzubringen. Vorzugsweise trifft die Nadelspitze der Vorstichhilfe mit einer Geschwindigkeit auf die Haut und durchschlägt diese, da sie aufgrund ihrer Massenträgheit und in Verbindung mit dem darunterliegenden Gewebe nicht so schnell nachgeben kann. Der Anschlag stoppt dann die Vorstichhilfe, um einen zu tiefen Vorstich zu verhindern.

Optional kann die Widerstandskomponente zumindest teilweise elastisch federnd verformbar und durch die distalwärtige manuelle Kraft spannbar sein.

Optional kann die Widerstandskomponente mindestens ein sich im Wesentlichen axial erstreckendes Federbein mit Fuß aufweisen, wobei der Fuß unter Spannung des Federbeins die distalwärtige manuelle Kraft radial weggedrückt wird und bei Überschreiten der maximalen Widerstandskraft unter schlagartiger Entspannung des Federbeins zurückschnappt.

Die Begriffe "distalwärtig' bzw. "proximalwärtig" sollen hierin eine relative Position bedeuten, die sich distal bzw. proximal von einem Anwender des Systems als Bezugsposition befindet. Die Begriffe "distalseitig" bzw. "proximalseitig" sollen hierin entsprechend Positionen an einer distalen bzw. proximalen Seite eines Gegenstands bedeuten. Die Begriffe "distalwärts" bzw. "proximalwärts" sollen hierin entsprechend Richtungen bedeuten, die sich nach distal bzw. proximal erstrecken.

Die Offenbarung ist nachfolgend anhand von in den Zeichnungen dargestellten Ausführungsbeispielen näher erläutert. Es zeigen:
Fig. 1 eine schematische Längsschnittdarstellung beim Einstechen eines Ausführungsbeispiels des hierin offenbarten Lichtapplikators in die Haut eines Patienten mit einem an der Haut platzierten Positionierungselement;
Figs. 2 bis 4 schematische Längsschnittdarstellungen eines Ausführungsbeispiels des hierein offenbarten Lichtapplikatorsystems in verschiedenen Stufen beim Vorstich der Haut mittels einer hierin offenbarten Vorstichhilfe;
Figs. 5a-c schematische Längsschnittdarstellungen verschiedener Ausführungsformen der Nadelspitze der hierein offenbarten Vorstichhilfe;
Figs. 6, 6a, 7 bis 11 schematische Längsschnittdarstellungen verschiedener Ausführungsformen des hierein offenbarten Lichtapplikatorsystems;
Figs. 12 bis 14 schematische Längsschnittdarstellungen einer Ausführungsform des hierein offenbarten Lichtapplikatorsystems in verschiedenen Stufen beim Vorstich der Haut zur Verdeutlichung des Problems bei sehr nachgiebiger Haut des Patienten; und
Fig. 15 bis 18 schematische Längsschnittdarstellungen einer Ausführungsform des hierein offenbarten Lichtapplikatorsystems in verschiedenen Stufen beim Vorstich der Haut zur Verdeutlichung der Lösung des Problems bei sehr nachgiebiger Haut des Patienten.

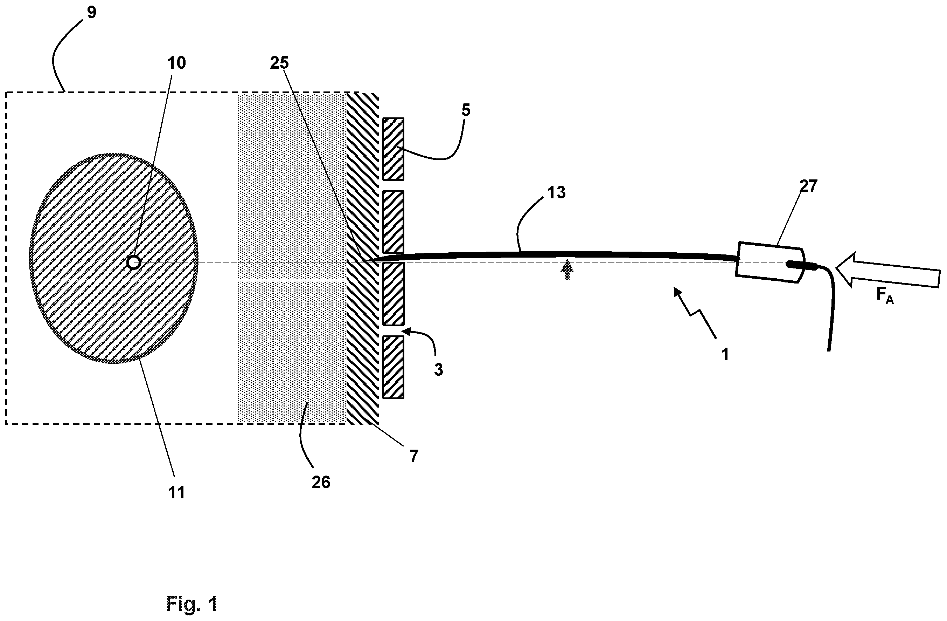

Fig. 1 verdeutlicht das mit dem hierin offenbarten Lichtapplikatorsystem gelöste Problem. Fig. 1 zeigt einen Lichtapplikator 1, der durch eine Aufnahme 3 eines Positionierungselements 5 in die Haut 7 eines organischen Körpers 9 gestochen wird. Das Positionierungselement 5 ist beispielsweise eine Einstichschablone, die derart positioniert auf die Haut 7 aufgeklebt ist, dass mit einem Durchstich durch die Aufnahme 3, die als Öffnung oder Loch in der Einstichschablone ausgebildet ist, ein genauer Zielpunkt 10 im Zielgewebe 11 im organischen Körper 9 mit dem Lichtapplikator 1 erreicht wird. Das Zielgewebe 11 kann beispielsweise ein Tumor in einem Organ oder das ganze Organ sein, beispielsweise die Prostata. Das Positionierungselement 5 kann, wie hier gezeigt, mehrere Aufnahmen 3 aufweisen, um mehrere Einstichmöglichkeiten zu bieten und/oder mehrere Lichtapplikatoren 1 gleichzeitig einsetzen zu können.

Ein distales Ende eines Einführabschnitts 13 des Lichtapplikators 1 soll dabei zur photodynamischen Therapie (PDT) oder Diagnose (PDD) in das Zielgewebe 11 eingestochen werden, um dieses Zielgewebe 11 mit Licht zu bestrahlen. Das distale Ende des Einführabschnitts 13 weist dazu ein aktiv lichtemittierendes Element in Form einer LED (hier nicht gezeigt) auf, deren Hauptabstrahlrichtung in Längsrichtung des Einführabschnitts 13 gerichtet ist. Zumindest teilweise distal von der LED ist eine sich distalwärts spitz verjüngende Nadelspitze 25 angeordnet, die einen lichttransparenten Streukörper zur Streuung des Lichts der LED aufweist. Die Nadelspitze 25 ist hier im Wesentlichen einstückig aus dem lichttransparenten Streukörper ausgebildet, der beispielweise Kunststoff mit Verstärkungselementen aufweisen kann.

Der Einführabschnitt 13 des Lichtapplikators 1 ist für eine möglichst minimalinvasive Behandlung oder Diagnose möglichst dünn (mit einem Durchmesser von 1 mm oder weniger) und starr ausgebildet. Allerdings muss der Einführabschnitt 13 des Lichtapplikators 1 zum Erreichen von relativ tief im organischen Körper 9 gelegenem Zielgewebe 11 relativ lang ausgebildet werden. Dies begrenzt die für den Einführabschnitt 13 des Lichtapplikators 1 erreichbare Biegesteifigkeit. Selbst bei Verwendung eines sehr biegesteifen, rohrförmig außenseitig angeordneten Schaftmaterials, beispielsweise Stahls, kann der Einführabschnitt 13 des Lichtapplikators 1 manuell verbogen werden beim Einstich in die Haut 7.

Dieses Problem ist in Fig. 1 gezeigt. Die Haut 7 kann sehr ledrig und zäh sein, sodass selbst bei sehr spitzer Nadelspitze 25 die aufzubringende manuelle Kraft F_{A}, mit der die Bedienperson ein proximalseitig am Einführabschnitt 13 des Lichtapplikators 1 angeordnetes Griffelement 27 des Lichtapplikators 1 distalwärts schiebt, relativ groß ist. Dabei kann sich der Einführabschnitt 13 des Lichtapplikators 1 wie gezeigt verbiegen. Eine solche Verbiegung ist allerdings selbst dann problematisch, wenn sie relativ klein ist, da die Nadelspitze 25 dadurch den Zielpunkt 10 im tiefliegenden Zielgewebe 11 verfehlen kann. Da die Eindringtiefe des Lichts in das Gewebe je nach verwendeter Wellenlänge nicht sehr tief ist und damit nur ein relativ kleines Gewebevolumen bestrahlt werden kann, müssen ggf. mehrere Lichtapplikatoren 1 zielgenau an einem jeweiligen Zielpunkt 10 im Zielgewebe 11 positioniert werden, um in der Summe der bestrahlten Gewebevolumina das gesamte Volumen des Zielgewebes 11 zu erfassen. Mit einer wie in Fig. 1 gezeigten Verbiegung des Einführabschnitts 13 des Lichtapplikators 1 kann eine zielgenaue Positionierung am Zielpunkt 10 im Zielgewebe 11 allerdings nicht sichergestellt werden.

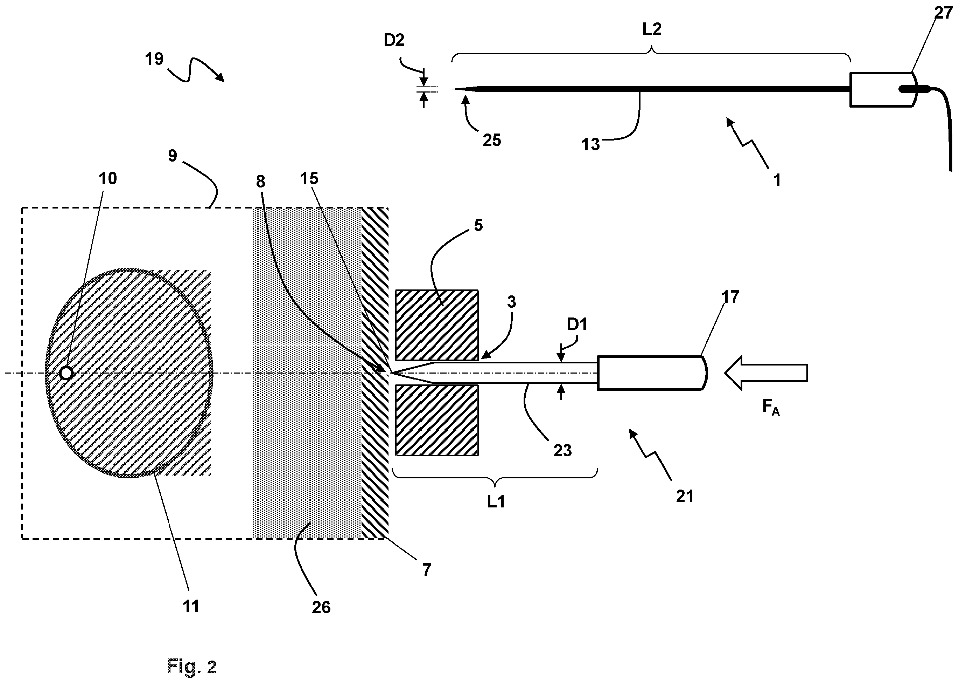

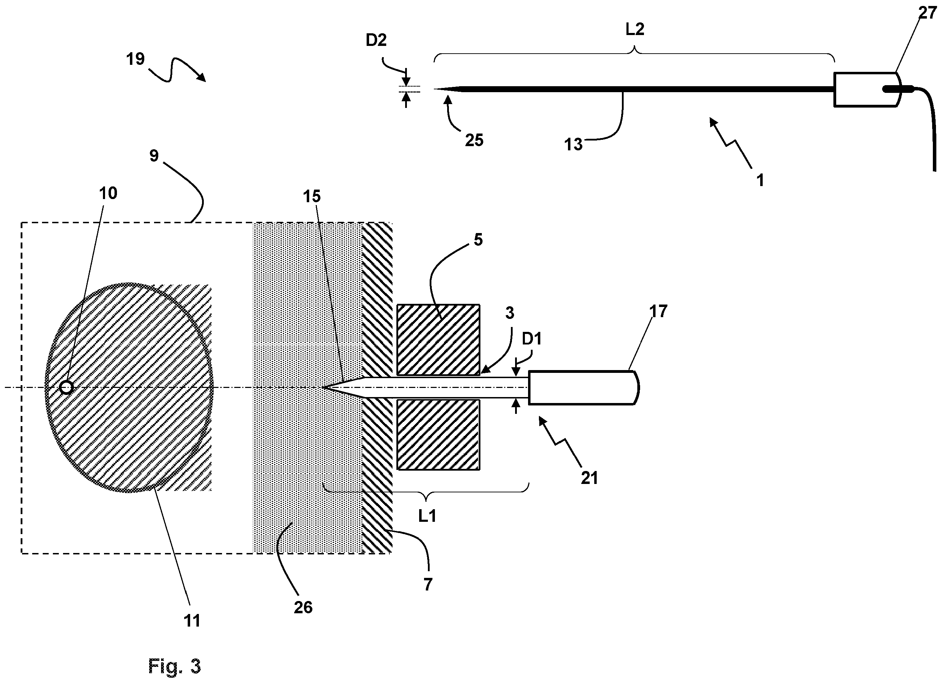

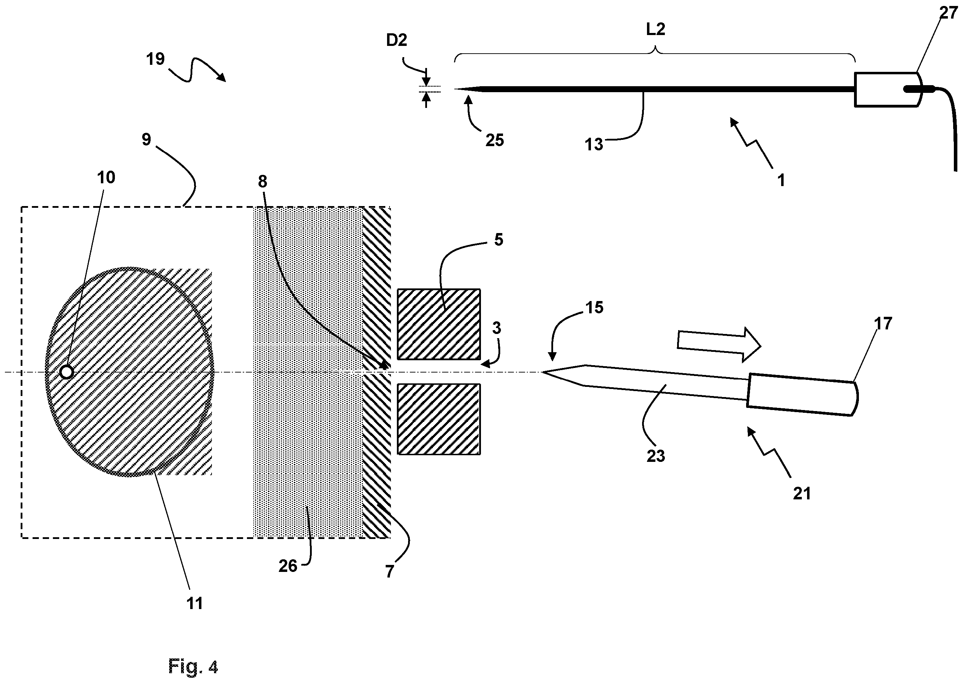

Figuren 2 bis 4 zeigen ein Lichtapplikatorsystem 19, das dieses Problem löst, ohne den Einführabschnitt 13 des Lichtapplikators 1 dicker und damit weniger minimalinvasiv auszugestalten. Das Lichtapplikatorsystem 19 weist dabei eine Vorstichhilfe 21 auf, die in der gezeigten Ausführungsform eine vom Lichtapplikator 1 separate Komponente ist. Bevor der Lichtapplikator 1 mit seiner in Bezug auf den Einführabschnitt 13 axial zentrisch angeordneten Nadelspitze 25 durch die Aufnahme 3 des Positionierungselements 5 in die Haut 7 gestochen wird, wird zunächst die Haut 7 zur Reduzierung des Hautwiderstands mittels der Vorstichhilfe 21 vorgestochen. Die Vorstichhilfe 21 ist im Wesentlichen in Form einer Nadel mit einem metallischen Vorstichhilfeschaft 23, einer am distalen Ende des Vorstichhilfeschafts 23 und in Bezug auf diesen Vorstichhilfeschaft 23 axial zentrisch angeordneten Nadelspitze 15 und einem am proximalen Ende des Vorstichhilfeschafts 23 angeordneten Griffelement 17 ausgestaltet, wobei der Vorstichhilfeschaft 23 ein wesentlich kleineres Länge-Durchmesser-Verhältnis hat als der Einführabschnitt 13 des Lichtapplikators 1, d.h. *L1*/*D1 «* L2/D2, und damit wesentlich biegesteifer. Vorzugsweise ist der Vorstichhilfeschaft 23 mit einer Länge L1 wesentlich kürzer als die Länge L2 des Einführabschnitts 13 des Lichtapplikators 1 und/oder mit einer Dicke D1 wesentlich dicker als die Dicke D2 des Einführabschnitts 13 des Lichtapplikators 1. Der Innendurchmesser der Aufnahme 3 des Positionierungselements 5 entspricht hier passgenau dem Außendurchmesser D1 des Vorstichhilfeschafts 23. Die sehr biegesteife Vorstichhilfe 21 ist durch die Aufnahme 3 des Positionierungselements 5 axial geführt und außerdem ist die Nadelspitze 15 der Vorstichhilfe 21 axial zentrisch angeordnet, sodass eine Position 8 des Vorstichs, d.h. die Position der von der Nadelspitze 15 erzeugten Öffnung 8 in der Haut 7, die später von der Nadelspitze 25 des Lichtapplikators 1 durch Vorschieben desselben innerhalb der Aufnahme 3 gefunden werden soll, sehr genau definiert ist. Die Vorstichhilfe 21 kann optional derart ausgestaltet sein, dass sie bei der in Fig. 3 gezeigten Axialposition, in der die Vorstichhilfe 21 die Haut 7 maximal aufgedehnt hat, mit einem hier nicht gezeigten Anschlag anstößt. Dadurch wird das unter der Haut 7 gelegene Gewebe 26 vor einem unnötig tiefen Vorstich mit der relativ dicken Vorstichhilfe 21 geschützt. In Fig. 4 ist gezeigt, wie die Vorstichhilfe 21 nach dem Vorstich proximalwärts aus der Aufnahme 3 des Positionierungselements 5 herausgezogen wird und eine an der genau definierten Position 8 vorgestochenen Haut 7, d.h. aufgrund der geforderten Minimalinvasivität lediglich eine kleine Öffnung hinterlässt, welche später, beim Einführen des Lichtapplikators 1 in den organischen Körper 9, von der Nadelspitze 25 des Lichtapplikators 1 dennoch zielgenau getroffen wird. Dabei ist es wichtig, dass das Positionierungselement 5 bezüglich der Haut 7 fest positioniert bleibt, beispielsweise aufgeklebt bleibt.

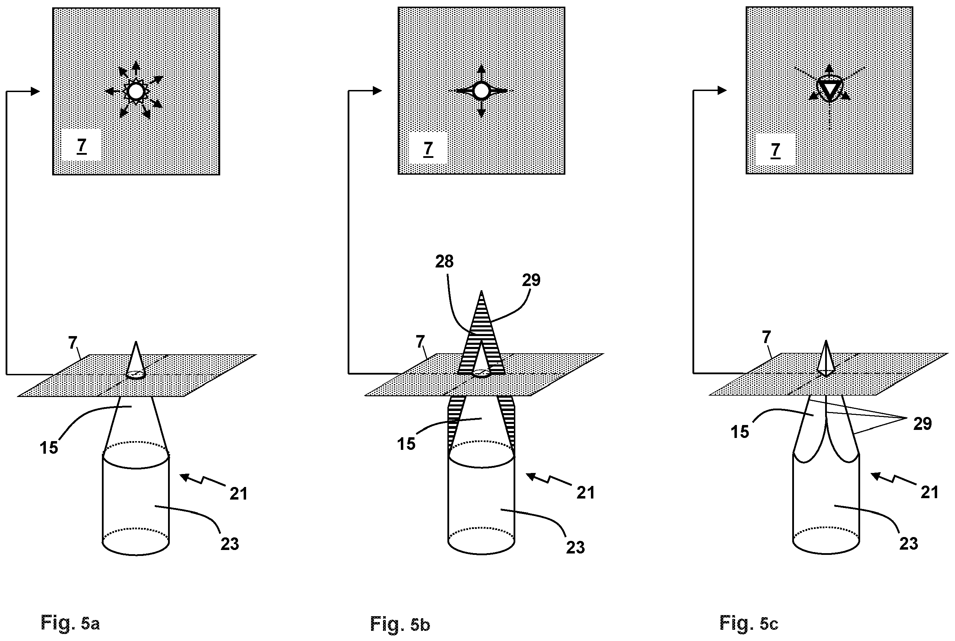

In Figuren 5a-c sind verschiedene Ausführungsformen der Nadelspitze 15 der Vorstichhilfe 21 gezeigt. In Fig. 5a ist die Nadelspitze 15 in der einfachsten Form kegelförmig ausgebildet, sodass die Haut 7 mit distalwärtigem Vordringen aufgeweitet wird. Um den Widerstand zu reduzieren, der sich aus der Dehnung der Haut 7 ergibt, weist die Nadelspitze 15 in Fig. 5b eine Schneidklinge 28 auf, die distal vorsteht und eine Schneidkante 29 bildet, welche die Haut 7 einschneidet. Dadurch wird weniger Hautspannung aufgebaut, sodass sich der Widerstand der Haut 7 reduziert. In Fig. 5c ist gezeigt, dass es mehrere Schneidkanten 29 geben kann, die nicht distal vorstehen müssen. Die Nadelspitze 23 ist in Fig. 5c pyramidenförmig, wobei die Pyramidenkanten die (hier drei) Schneidkanten 29 bilden.

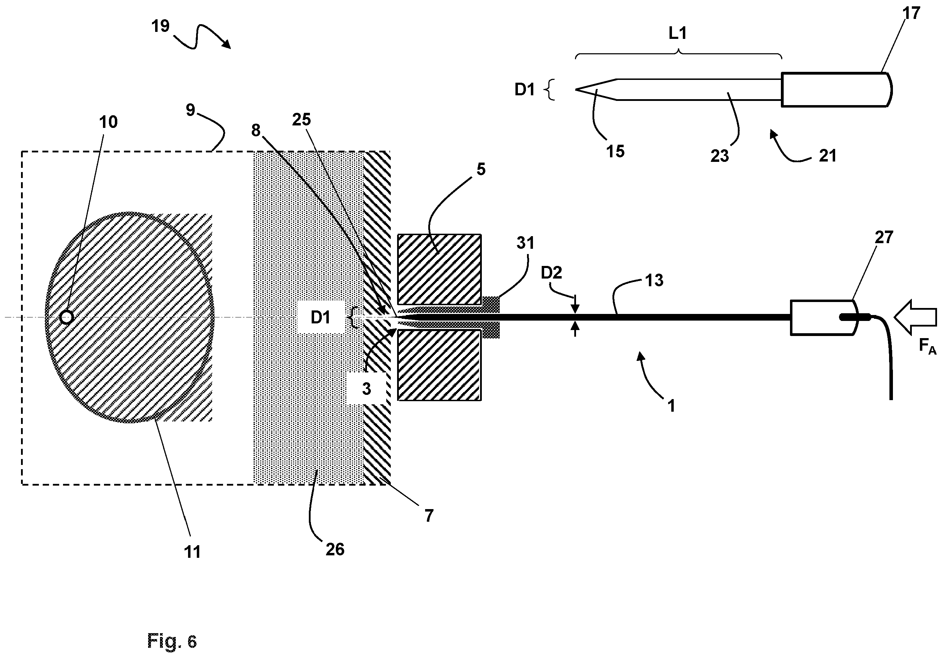

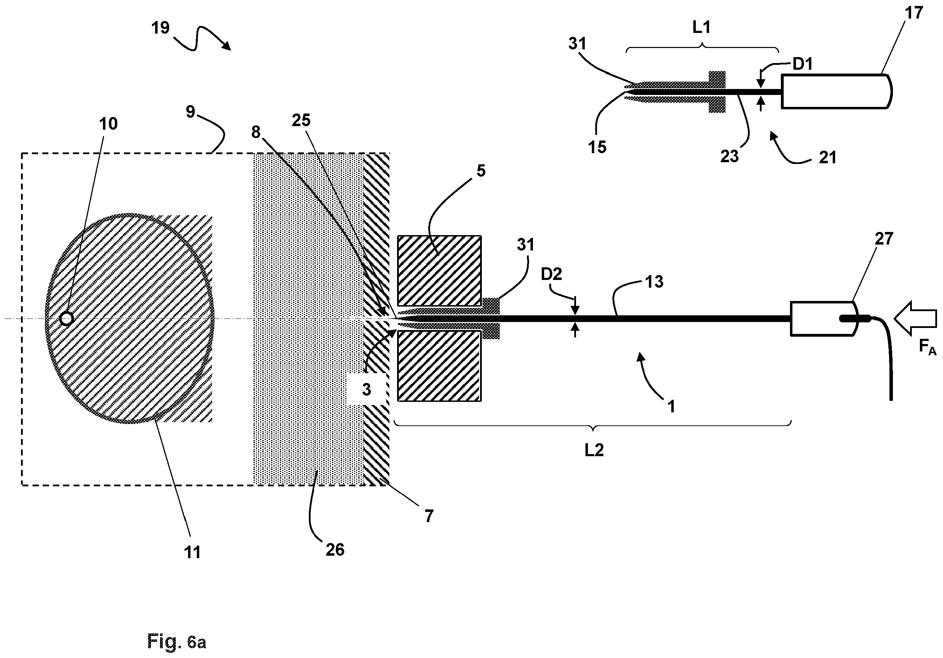

Fig. 6 zeigt, wie der Lichtapplikator 1 nach dem Vorstich mittels der nunmehr aus dem Positionierungselement 5 entfernten Vorstichhilfe 21 verwendet werden kann. Da der Einführabschnitt 13 des Lichtapplikators 1 wesentlich dünner als die Vorstichhilfe 21 ist, ist eine Adapterhülse 31 vorgesehen, die den Unterschied zwischen der Dicke D1 des Vorstichhilfeschafts 23 und der Dicke D2 des Einführabschnitts 13 des Lichtapplikators 1 dadurch ausgleicht, sodass der Innendurchmesser der Adapterhülse 31 passgenau dem Außendurchmesser D2 des Einführabschnitts 13 des Lichtapplikators 1 entspricht und die Adapterhülse 31 zumindest abschnittsweise einen Außendurchmesser D1 aufweist, der passgenau in einen entsprechenden Innendurchmesser der Aufnahme 3 des Positionierungselements 5 passt.

Die Adapterhülse 31 kann als Schutzhülse für die empfindliche Nadelspitze 25 des Lichtapplikators 1 dienen und unverlierbar axial beweglich am Einführabschnitt 13 des Lichtapplikators 1 vorinstalliert sein. Alternativ dazu kann die Adapterhülse 31 zunächst manuell in die Aufnahme 3 eingesteckt werden und dann der Einführabschnitt 13 des Lichtapplikators 1 eingeführt werden, oder es kann die Adapterhülse 31 zunächst auf den Einführabschnitt 13 des Lichtapplikators 1 aufgesteckt und dann zusammen mit diesem in die Aufnahme 3 eingesteckt werden. Die Adapterhülse 31 kann zudem als Stabilisierungshülse für den Einführabschnitt 13 des Lichtapplikators 1 dienen, um zumindest abschnittsweise eine wie in Fig. 1 gezeigte Verbiegung des Einführabschnitts 13 des Lichtapplikators 1 zu verhindern.

Durch den Vorstich der Haut 7 bzw. die dadurch erzeugte Öffnung 8 mittels der Vorstichhilfe 21 bzw. deren axial zentrisch angeordneten Nadelspitze 15 ist allerdings die Widerstandskraft der Haut 7 bereits so stark reduziert, dass die Bedienperson nur eine geringe manuelle Kraft F_{A} auf den Lichtapplikator 1 ausüben muss, um den Einführabschnitt 13 des Lichtapplikators 1 in den Körper 9 einzustechen. Daher ist das Risiko einer Verbiegung bereits stark reduziert. Das Positionierungselement 5 sowie die gleichermaßen axial zentrische Anordnung der Nadelspitze 25 des Lichtapplikators 1 stellen dabei sicher, dass die Nadelspitze 25 des Lichtapplikators 1 beim Vorschieben desselben innerhalb der Aufnahme 3 zielgenau den mittels der Vorstichhilfe 21 bzw. den mit ihrer axial zentrisch angeordneten Nadelspitze 15 vorgestochenen Hautpunkt 8 trifft, um so den auf den Lichtapplikator 1 wirkenden Widerstand durch die Haut zu reduzieren. Um eine ausreichend genaue axiale Führung bereitzustellen, damit einerseits der definierte Hautpunkt 8 von beiden Nadelspitzen 15 und 25 gleichermaßen zielgenau erreicht wird und andererseits das relativ tief gelegene Zielgewebe 11 zielgenau mit der Nadelspitze 15 des Lichtapplikators 1 getroffen wird, weist das Positionierungselement 5 vorzugsweise eine gewisse Dicke in Axialrichtung der Aufnahme 3 auf. Das heißt, die Aufnahme 3 hat eine ausreichende Länge, um eine gute Führungsgenauigkeit zu erzielen.

Fig. 6a zeigt ein Lichtapplikatorsystem 19, bei dem sowohl die Nadelspitze 25 des Lichtapplikators 1 als auch die Nadelspitze 15 der Vorstich hilfe 21 jeweils in eine eigene Adapterhülse 31 eingebracht sind, welche u.a. zum Schutz der Bedienperson, des Positionierungselements 5 und der jeweiligen Nadelspitze 15 bzw. 25 selbst dient, und zwar so lange, bis diese distalseitig die Aufnahme 3 des Positionierungselements verlässt. Dazu kann die Adapterhülse 31 bereits vorab unverlierbar und axial beweglich am Vorstichhilfeschaft 23 bzw. am Einführabschnitt 13 des Lichtapplikators 1 angebracht sein, wobei sich die jeweilige Nadelspitze 15 bzw. 25 vorzugsweise erst dann aus ihrer Adapterhülse 31 herausbewegen kann, wenn die Adapterhülse 31 ihre finale Position in der Aufnahme 3 des Positionierungselements 5 erreicht hat. Dabei sind Im vorliegenden Ausführungsbeispiel der Durchmesser D1 des Vorstichhilfeschafts 23 und der Durchmesser D2 des Einführabschnitts 13 des Lichtapplikators 1 gleich groß, so dass die Adapterhülse 31 für den Lichtapplikator 1 und die Adapterhülse 31 für die Vorstichhilfe 21 baugleich ausgeführt werden können. Der Innendurchmesser der Adapterhülse 31 ist an den Außendurchmesser D1 bzw. D2 des Vorstichhilfeschafts 23 bzw. des Einführabschnitts 13 angepasst und der Außendurchmesser der Adapterhülse 31 entspricht zumindest abschnittsweise dem Innendurchmesser der Aufnahme 3 des Positionierungselements 5. Da nun erstens die Adapterhülse 31 bzgl. ihrer Geometrie (Durchmesser) in der vorausgehend beschriebenen Weise an den Einführabschnitt 13 des Lichtapplikators 1, den Vorstichhilfeschaft 23 und die Aufnahme 3 angepasst ist und da zudem sowohl die Nadelspitze 25 des Lichtapplikators 1 in Bezug auf die Achse des Einführabschnitts 13 des Lichtapplikators 1 zentrisch angeordnet ist als auch die Nadelspitze 15 der Vorstichhilfe 21 in Bezug auf die Achse des Vorstichhilfeschafts 23 zentrisch angeordnet ist, ist sichergestellt, dass die Nadelspitze 25 des Lichtapplikators 1 nach dem Einführen und Vorschieben in die Aufnahme 3 mit ihrer vorab aufgebrachten Adapterhülse 31 automatisch bzw. von selbst die bereits vorab von der Nadelspitze 15 der Vorstichhilfe 21 in der Haut 7 erzeugte Öffnung 8 findet, um so den von der zähen Haut 7 verursachten Widerstand, den der Lichtapplikator 1 beim Einführen bzw. Vorschieben in bzw. durch die Haut 7 erfährt, deutlich zu reduzieren. Die gegenüber dem Lichtapplikator 1 erhöhte Biegesteifigkeit der Vorstichhilfe 21 wird in diesem Ausführungsbeispiel dadurch erreicht, dass der Vorstichhilfeschaft 23 deutlich kürzer als der Einführabschnitt 13 des Lichtapplikators 1 ausgeführt wird, d.h. L1 << L2. Auch hier hat die Aufnahme 3 hat eine ausreichende Länge, um eine gute Führungsgenauigkeit von Lichtapplikator 1 und Vorstichhilfe 21 zu erzielen.

Eine weitere Ausführungsform (ohne Abbildung) orientiert sich an der in Fig. 6a dargestellten Ausführungsform. Allerdings wird dabei auf die Adapterhülsen 31 sowohl bei der Vorstichhilfe als auch beim Lichtapplikator 1 verzichtet. Um allerdings trotzdem auch hier die gewünschte Führungsgenauigkeit von Vorstichhilfe 21 und Lichtapplikator 1 zu erreichen als Voraussetzung dafür, dass erstens sowohl die Vorstichhilfe 21 als auch der Lichtapplikator 1 - im Sinne einer Widerstandsreduzierung für den Lichtapplikator 1 an der Haut 7 - gleichermaßen die definierte Position der Öffnung 8 in der Haut 7 erreichen und zweitens der Lichtapplikator 1 dann den Zielpunkt 10 im organischen Körper 9 erreicht, wird nun, im Gegensatz zur Ausführungsform der Fig. 6, der Innendurchmesser der Aufnahme 3 an die Außendurchmesser D1 bzw. D2 von Vorstichhilfeschaft 23 bzw. Einführabschnitt 13 des Lichtapplikators 1, die in dieser Ausführungsform gleich groß sind, angepasst.

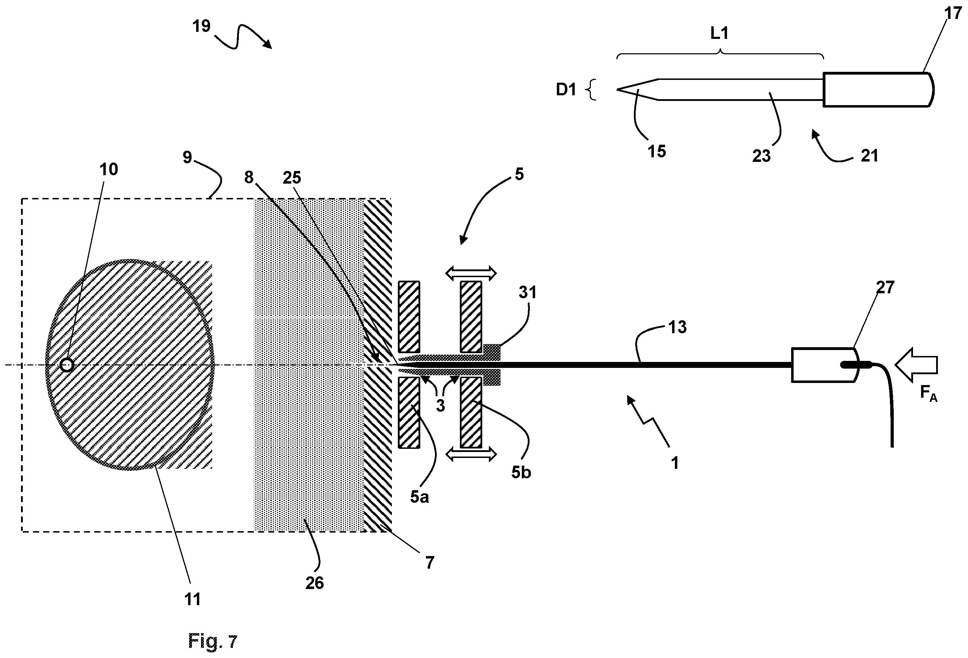

Wenn das Positionierungselement 5, beispielsweise als Lochschablone, mehrere Aufnahmen 3 für die gleichzeitige Aufnahme mehrerer Lichtapplikatoren 1 ist, haben die in Fig. 6 und Fig. 6a gezeigten Ausführungsbeispiele den Nachteil, dass das Positionierungselement 5 relativ voluminös und ggf. schwer ausgestaltet ist. Dies kann zwar akzeptabel oder auch vorteilhaft für bestimmte Anwendungen sein, aber für die meisten Anwendungen ist es nachteilhaft. In Fig. 7 ist ein Ausführungsbeispiel des Positionierungselements 5 gezeigt, bei dem das Positionierungselement 5 zwei axial auf Abstand gehaltene Positionierungselementkomponenten 5a,5b aufweist, von denen jede mindestens eine Aufnahme 3 aufweist, die mit einer entsprechenden Aufnahme 3 der anderen Komponente axial fluchtet. Vorzugsweise ist der axiale Abstand zwischen den Positionierungselementkomponenten 5a,5b einstellbar, wie in der Fig. 7 schematisch dargestellt. Je größer der Abstand ist, umso besser ist die Führungsgenauigkeit. Ein kleinerer Abstand kann für die Handhabung bei der Positionierung des Positionierungselements 5 relativ zum organischen Körper 9 sinnvoll sein. Im Gegensatz zu den Ausführungsbeispielen gemäß Fig. 6 und 6a kann eine entsprechende Führungsgenauigkeit mit weniger Volumen und Gewicht des Positionierungselements 5 erzielt werden.

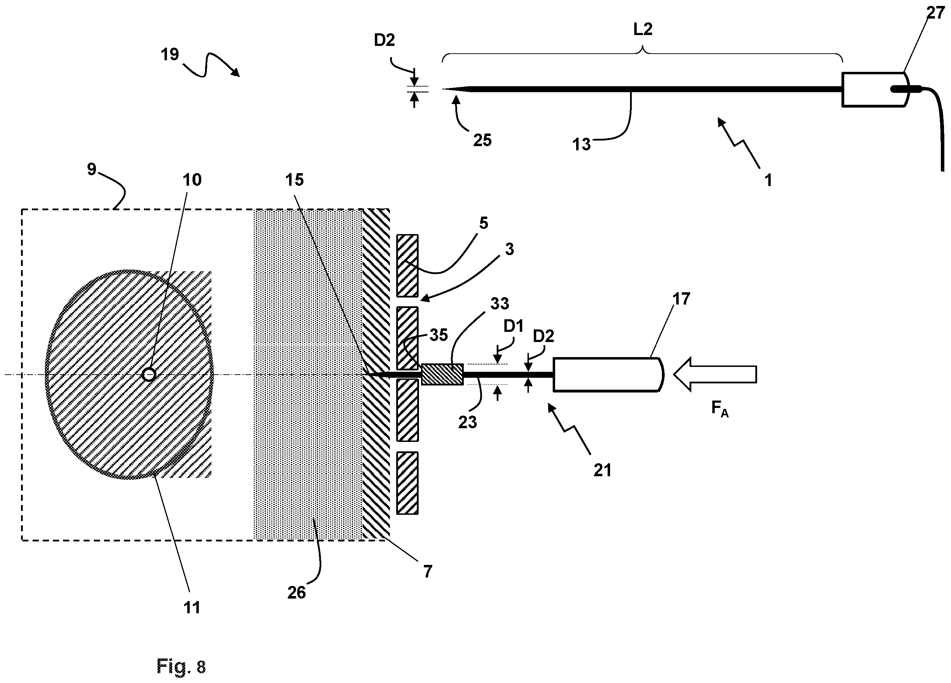

Fig. 8 zeigt eine Ausführungsform der Vorstichhilfe 21, bei welcher der Vorstichhilfeschaft 23 nur abschnittsweise in einem Verdickungsabschnitt 33 eine Dicke D1 aufweist, die dicker als die Dicke D2 des Einführabschnitt 13 des Lichtapplikators 1 ist und dadurch für den Vorstich der Haut 7 eine höhere Biegesteifigkeit erreicht wird. Distal vom Verdickungsabschnitt 33 entspricht der Außendurchmesser des Vorstichhilfeschafts 23 der Dicke D2 des Einführabschnitt 13 des Lichtapplikators 1. Dies hat den Vorteil, dass die Aufnahme 3 im Positionierungselement 5 mit einem Innendurchmesser D2 dünner ausgestaltet werden kann, sodass mehr Aufnahmen 3 pro Flächeneinheit im Positionierungselement 5 untergebracht werden können, was für eine engmaschige Positionierung von mehreren parallelen Lichtapplikatoren 1 vorteilhaft ist. Außerdem wird für die Lichtapplikatoren 1 keine Adapterhülse 31 benötigt. Ein distales Ende des Verdickungsabschnitts 33 kann als Anschlag 35 gegen das Positionierungselement 5 stoßen, wenn eine gewünschte Vorstichtiefe durch die Haut 7 erreicht ist.

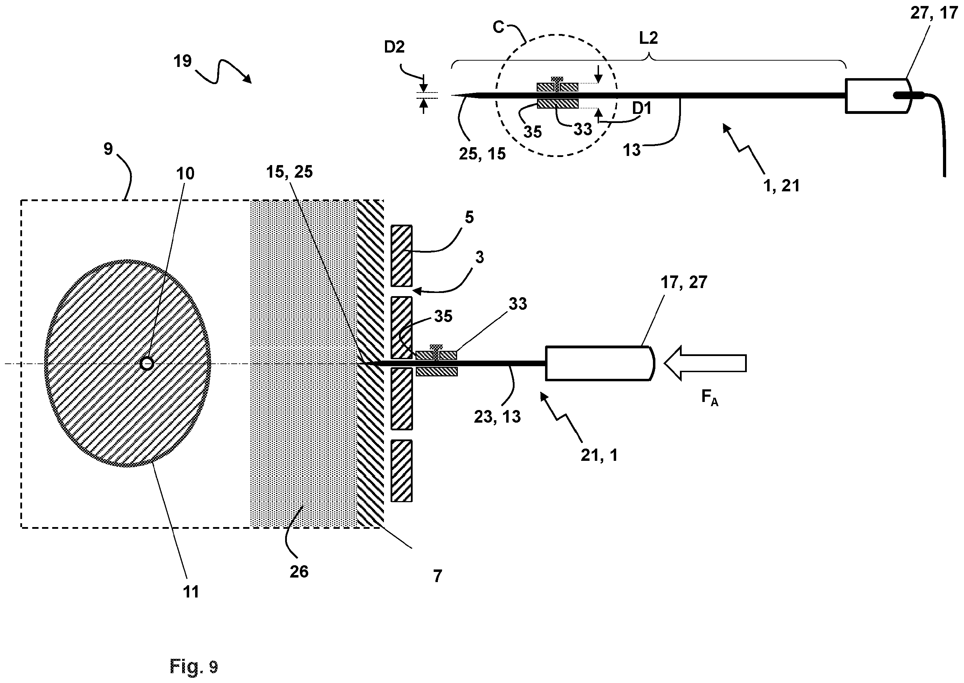

In Fig. 9 ist durch einen gestrichelten Kreis C eine Ausführungsform der Erfindung angedeutet, bei der die Vorstichhilfe 21 keine separate Komponente ist, sondern in den Lichtapplikator integriert ist. Der Verdickungsabschnitt 33 kann beispielsweise durch eine auf dem Einführabschnitt 13 des Lichtapplikators 1 axial positionier- und fixierbare Stabilisierungshülse mit einem Außendurchmesser D1 realisiert sein. In diesem Fall wird der Vorstichhilfeschaft 23 durch den Einführabschnitt 13 des Lichtapplikators 1 gebildet, und die Nadelspitze 25 des Lichtapplikators 1 ist die Nadelspitze 15 der Vorstichhilfe 21. Die Stabilisierungshülse 33 erhöht dabei lokal die Biegesteifigkeit des Einführabschnitts 13 des Lichtapplikators 1, sodass dieser sich beim Vorstich in die Haut nicht oder nur unwesentlich verbiegt. Die Stabilisierungshülse 33 kann beispielsweise mittels einer Klemmschraube oder -backe an einer gewählten Axialposition auf dem Vorstichhilfeschaft 23 fixiert werden, um die Anschlagsposition 35 und damit die Vorstichtiefe einzustellen und an den Anwendungsfall anzupassen.

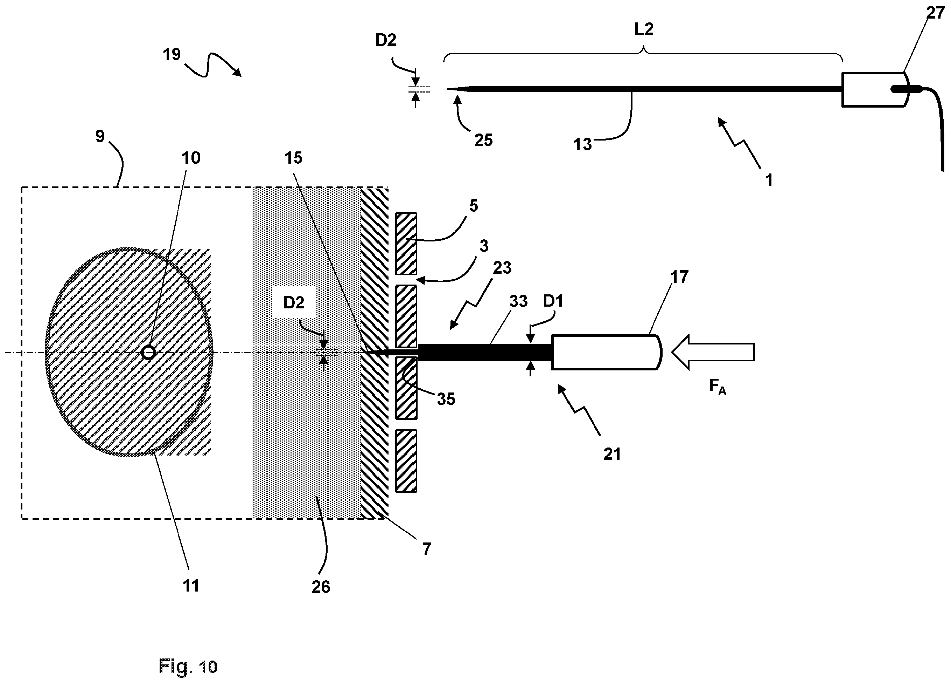

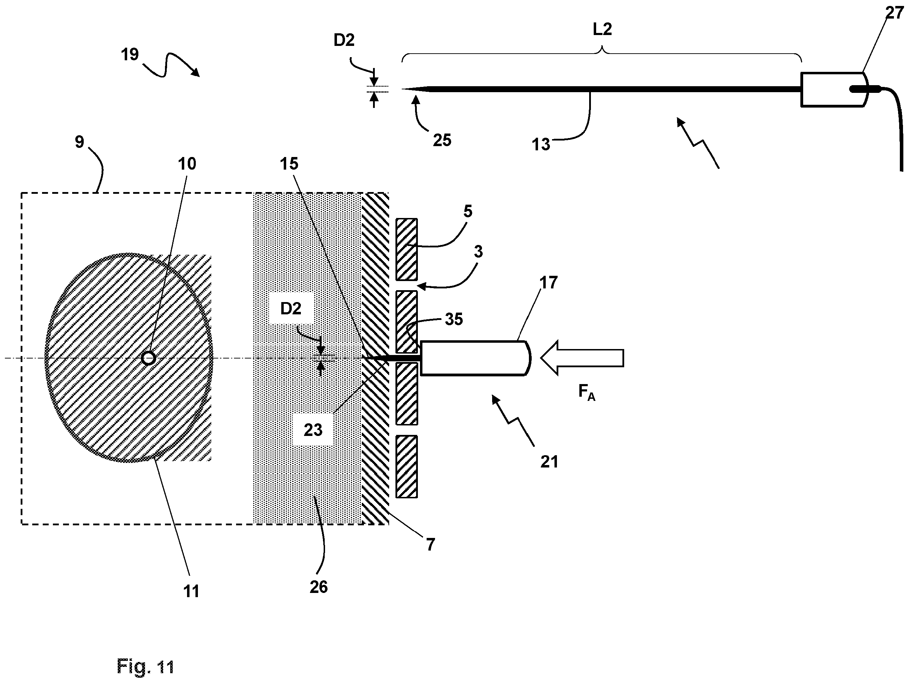

Fig. 10 und 11 zeigen unterschiedliche Ausführungsformen für die Realisierung des Anschlags 35. In Fig. 8 und 9 bildet ein distales Ende des Verdickungsabschnitts 33 den Anschlag 35, der gegen das Positionierungselement 5 stößt, wenn eine gewünschte bzw. eingestellte Vorstichtiefe durch die Haut 7 erreicht ist. In Fig. 10 weist ein distaler Abschnitt des Vorstichhilfeschafts 23 einen dünneren Durchmesser D2 gegenüber einem proximalen Abschnitt des Vorstichhilfeschafts 23 auf, der einen dickeren Durchmesser D1 hat. Der proximale Abschnitt des Vorstichhilfeschafts 23 fungiert also als Verdickungsabschnitt 33, dessen distales Ende den Anschlag 35 bildet. Dies ist für die Biegesteifigkeit des Vorstichhilfeschafts 23 vorteilhaft. Das Ausführungsbeispiel gemäß Fig. 11 kommt ganz ohne den proximalen Abschnitt des Vorstichhilfeschafts 23 aus, wobei der Vorstichhilfeschafts 23 so kurz ist, dass er nur aus dem distalen Abschnitt mit dem dünnen Durchmesser D2 besteht. Der Anschlag 35 wird hier durch das Griffelement 17 gebildet. Die Kürze des Vorstichhilfeschafts 23 ist für die Biegesteifigkeit vorteilhaft.

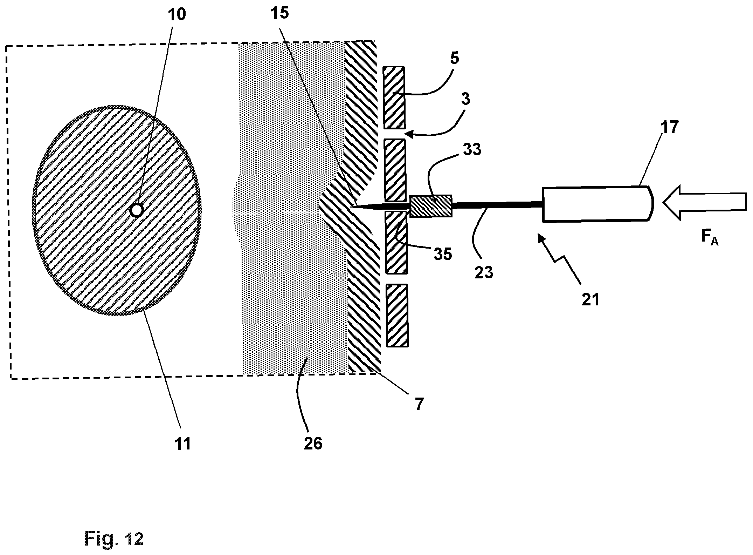

Fig. 12 bis 14 zeigen eine Problematik bei besonders nachgiebiger und/oder ledriger Haut 7. Beim Vorstich kann die Haut 7 nämlich ggf. wie in Fig. 12 gezeigt distalwärts nachgeben. Dieses Risiko wird bereits dann reduziert, wenn dass das Positionierungselement 5 auf die Haut 7 geklebt ist. Allerdings kann sich die Haut 7 selbst dann distalwärts ablösen und eindellen, anstatt sich für den Vorstich der Nadelspitze 15 der Vorstichhilfe 21 zu öffnen. Wenn dies, wie in Fig. 12 gezeigt, bis zum Anschlag 35 geschieht, hat der Vorstich so gut wie keine Wirkung. Da das Positionierungselement 5 die Sicht auf die Vorstichstelle versperrt, bemerkt die Bedienperson die Wirkungslosigkeit des Vorstichs ggf. nicht. Dazu kann das Positionierungselement 5 zwar zumindest um die Aufnahme 3 herum vorzugsweise transparent ausgestaltet werden, aber aufgrund von Reflexionen an optischen Grenzflächen kann eine Sicht auf die Vorstichstelle versperrt bleiben.

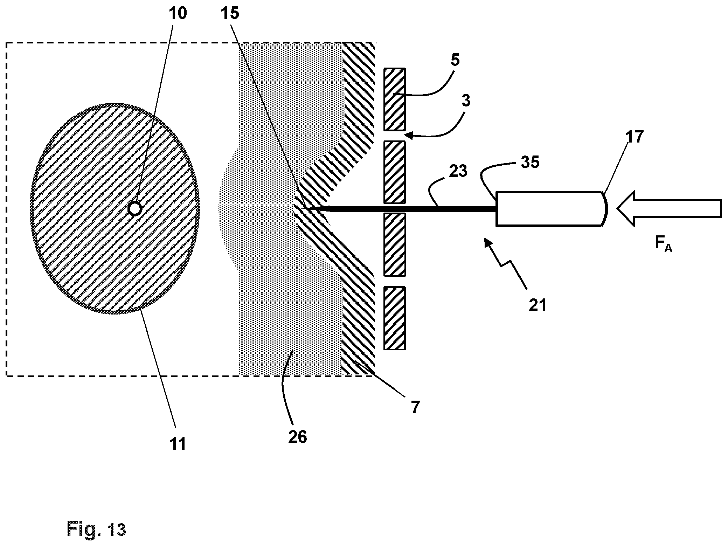

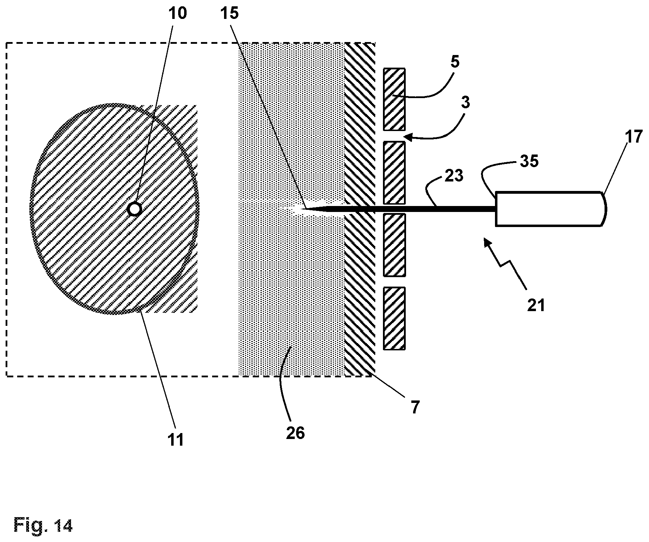

Da sich die Haut 7 während des (nicht erfolgreichen) Vorstichs dehnt, wird die Reaktionskraft der Haut 7 und der entsprechende Druck, den die Nadelspitze 15 auf die Haut 7 ausübt, distalwärts größer. Wenn man nun wie in Fig. 13 versucht, die Vorstichtiefe durch proximalwärtige Verschiebung des Anschlags 35 zu vergrößern, um die für einen erfolgreichen Vorstich benötigte Reaktionskraft der Haut 7 zu erreichen, kann die in Fig. 14 gezeigte unerwünschte Situation eintreten. Die Haut 7 öffnet sich ab einer bestimmten Vorstichtiefe schlagartig und schnellt proximalwärts zurück. Dabei sticht die Nadelspitze 15 wegen der größeren Vorstichtiefe in unter der Haut 7 befindliches Gewebe 26, in das die Vorstichhilfe 21 eigentlich nicht vorgestochen werden sollte. Der Vorstich ist damit weniger minimalinvasiv als gewünscht.

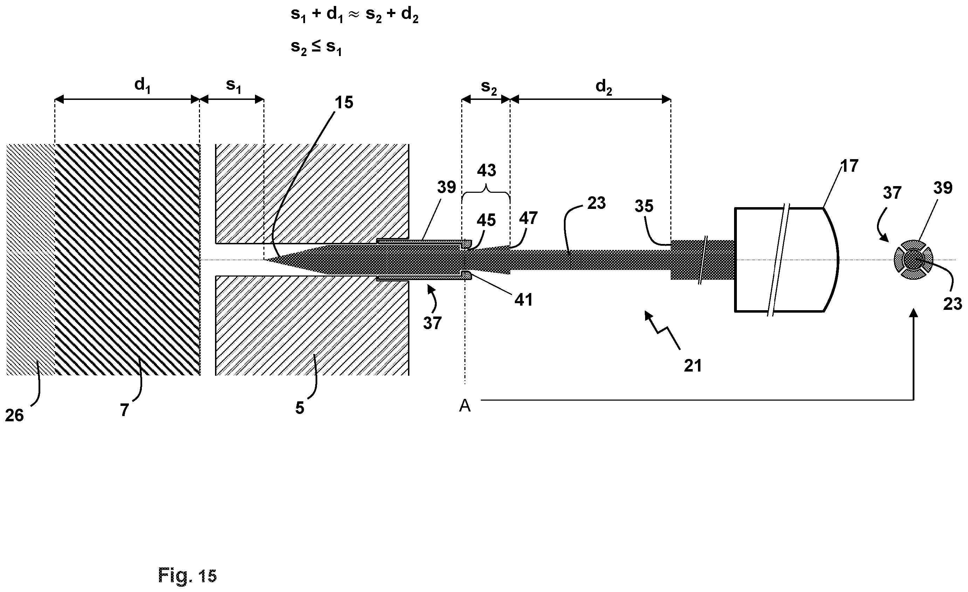

Das in Fig. 15 bis 18 gezeigte Ausführungsbeispiel löst das in Fig. 12 bis 14 gezeigte Problem besonders nachgiebiger und/oder ledriger Haut 7. Dazu ist eine Widerstandskomponente 37 vorgesehen, um von der Haut 7 die Funktion des Aufbauens einer Reaktionskraft zu übernehmen, um den für den Vorstich notwendigen Druck auf die Haut 7 aufzubauen. Die Widerstandskomponente 37 ist hier eine am Positionierungselement 5 angeordnete von proximalseitig axial eingeschlitzte Hülse, die federnd nach radial außen nachgebende Federbeine 39 mit sich radial nach innen erstreckenden Füßen 41 bildet. Der Vorstichhilfeschaft 23 weist einen axialen Abschnitt 43 auf, dessen Außerdurchmesser sich graduell und/oder schrittweise von einem distalen Ende 45 proximalwärts zu einem maximalen Durchmesser an einem proximalen Ende 47 des Abschnitt 43 vergrößert. Proximal vom proximalen Ende 47 des Abschnitts 43 ist der Vorstichhilfeschaft 23 wieder dünner. In Fig. 15 ist eine Axialposition beim Beginn des Vorstichs gezeigt, bei der die Federbeine 39 der Widerstandskomponente 37 entspannt axial ausgerichtet sind und die Füße 41 am distalen Ende 45 des Abschnitts 43 am Vorstichhilfeschaft 23 anliegen. Das distale Ende der Nadelspitze 15 der Vorstichhilfe 21 hat dabei noch einen axialen Abstand s₁ zur Haut 7, der mindestens der axialen Länge s₂ des Abschnitts 43 entspricht.

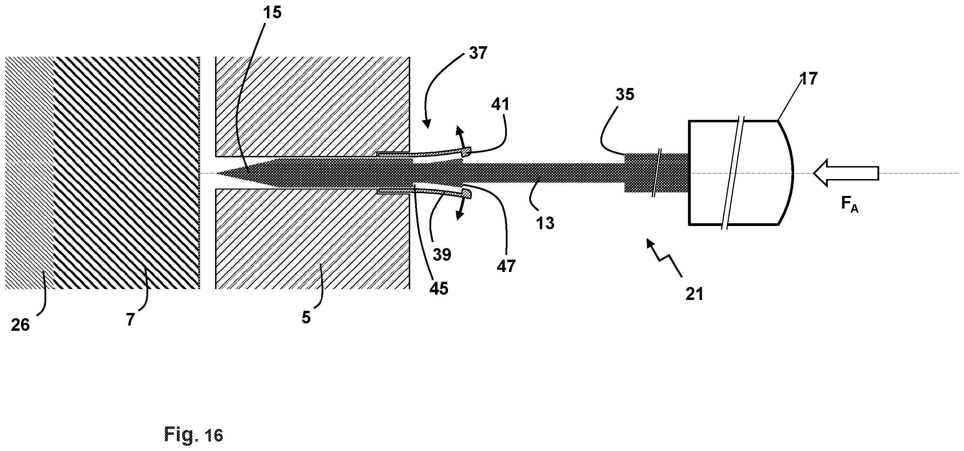

Wird nun, wie in Fig. 16 gezeigt, die Vorstichhilfe 21 distalwärts geschoben, verbiegen sich die Federbeine 39 gegen ihre nach radial innen gerichtete Rückstellkraft nach außen, da die Füße 41 durch den sich vergrößernden Durchmesser radial nach außen gedrückt werden. Dafür muss die Bedienperson eine distalwärts gerichtete manuelle Kraft auf das Griffelement 17 ausüben. Diese ist bei in Fig. 16 gezeigter maximaler Spannung der Federbeine 39 maximal, wenn die Füße 41 das distale Ende 47 des Anschnitts 43 erreicht haben.

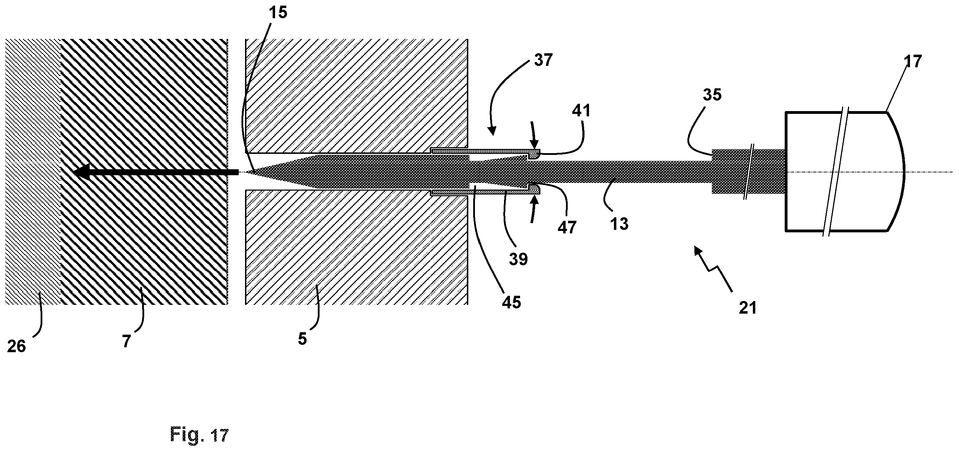

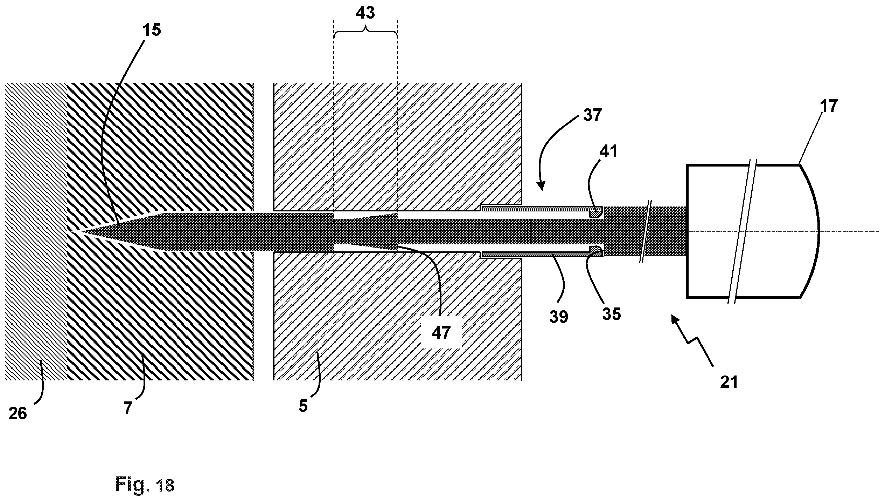

Sobald die Vorstichhilfe 21 über den in Fig. 16 gezeigten Punkt distalwärts geschoben wird, schnappen die Federbeine 39, wie in Fig. 17 gezeigt, schlagartig zurück in ihre entspannte axiale Ausrichtung. Die noch wirkende manuelle Kraft der Bedienperson führt nun zu einem Kraftstoß mit entsprechender distalwärtiger Beschleunigung der Vorstichhilfe 21. Die Nadelspitze 15 der Vorstichhilfe 21 trifft also mit diesem Kraftstoß auf die Haut 7, welche aufgrund ihrer Trägheit nicht so schnell nachgeben kann und der Druck augenblicklich so groß ist, dass sich die Haut 7 für den Vorstich öffnet, wie in Figur 18 gezeigt.

Der Vorstichhilfeschaft 23 bildet proximal vom proximalen Ende 47 des Abschnitts 43 einen Anschlag 35 für die Füße 41 der Federbeine 39, sodass die Vorstichhilfe 21 bei der vorgesehenen Vorstichtiefe dadurch gestoppt wird, dass die Füße 41 an den Anschlag 35 schlagen. Der Abstand d₂ des Anschlags 35 vom proximalen Ende 47 des Abschnitts 43 entspricht hier in etwa der Dicke d₁ der Haut 7, damit diese vollständig durchstochen wird, aber die Nadelspitze 15 nicht oder nur sehr wenig in das unter der Haut 7 liegende Gewebe 26 gestochen wird. Wenn die Nadelspitze 15 bereits mit Geschwindigkeit auf die Haut 7 treffen soll, also *s*₁ > *s*₂ ist, dann ist vorzugsweise entsprechend *d*₂ *> d*₁, damit *s*₁ + *d*₁ ≈ *s*₂ *+ d*₂ gilt.

Die Widerstandskomponente 37 kann Teil des Positionierungselements 5 sein oder an dieser angebracht oder anbringbar sein. Alternativ dazu kann die Widerstandskomponente 37 Teil der Vorstichhilfe 21 sein und/oder an dieser angebracht oder anbringbar sein. Vorzugsweise kann sie unverlierbar als axial bewegliche Hülse am Vorstichhilfeschaft 23 angebracht sein.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden. Eine Bezeichnung eines Bauteils oder technischen Merkmals als "erstes" soll nicht dahingehend missverstanden werden, dass es ein zweites Bauteil oder technisches Merkmal dieser Art geben muss. Außerdem können etwaige Verfahrensschritte, soweit nicht explizit anders erläutert oder zwingend erforderlich, in beliebiger Reihenfolge und/oder zeitlich teilweise oder ganz überlappend durchgeführt werden.

Äquivalente Ausführungsformen der hierin beschriebenen Parameter, Bauteile oder Funktionen, die in Anbetracht dieser Beschreibung einer fachlich versierten Person als offensichtlich erscheinen, seien hierin so erfasst als wären sie explizit beschrieben. Entsprechend soll der Schutzbereich der Ansprüche solche äquivalente Ausführungsformen umfassen. Als optional, vorteilhaft, bevorzugt, erwünscht oder ähnlich bezeichnete "kann"-Merkmale sind als optional zu verstehen und nicht als schutzbereichsbeschränkend.

Die beschriebenen Ausführungsformen sind als illustrative Beispiele zu verstehen und stellen keine abschließende Liste von möglichen Ausführungsformen dar. Jedes Merkmal, das im Rahmen einer Ausführungsform offenbart wurde, kann allein oder in Kombination mit einem oder mehreren anderen Merkmalen verwendet werden, unabhängig davon, in welcher Ausführungsform die Merkmale jeweils beschrieben wurden. Während mindestens ein Ausführungsbeispiel hierin beschrieben und gezeigt ist, seien Abwandlungen und alternative Ausführungsformen, die einer fachmännisch versierten Person in Anbetracht dieser Beschreibung als offensichtlich erscheinen, vom Schutzbereich dieser Offenbarung mit erfasst. Im Übrigen soll hierin weder der Begriff "aufweisen" zusätzliche andere Merkmale oder Verfahrensschritte ausschließen noch soll "ein" oder "eine" eine Mehrzahl ausschließen.

### Bezugszeichenliste:

- 1: Lichtapplikator
- 3: Aufnahme
- 5: Positionierungselement
- 7: Haut
- 8: Position des Vorstichs, Öffnung in der Haut, vorgestochener Hautpunkt
- 9: organischer Körper
- 10: Zielpunkt
- 11: Zielgewebe
- 13: Einführabschnitt
- 15: Nadelspitze der Vorstichhilfe
- 17: Griffelement der Vorstichhilfe
- 19: Lichtapplikatorsystem
- 21: Vorstichhilfe
- 23: Vorstichhilfeschaft
- 25: Nadelspitze des Lichtapplikators
- 26: unter der Haut liegendes Gewebe
- 27: Griffelement des Lichtapplikators
- 28: Schneidklinge
- 29: Schneidkante(n)
- 31: Adapterhülse
- 33: Verdickungsabschnitt / Stabilisierungshülse
- 35: Anschlag
- 37: Widerstandskomponente
- 39: Federbeine
- 41: Füße
- 43: Abschnitt des Vorstichhilfeschaft
- 45: distales Ende des Abschnitts des Vorstichhilfeschafts
- 47: proximales Ende des Abschnitts des Vorstichhilfeschafts
- D1: Durchmesser des Vorstichhilfeschafts
- L1: Länge des Vorstichhilfeschafts
- D2: Durchmesser des Einführabschnitts des Lichtapplikators
- L2: Länge des Einführabschnitts des Lichtapplikators
- d₁: Dicke der Haut
- s₁: Abstand der Nadelspitze der Vorstichhilfe zur Haut
- d₂: Abstand des Anschlags vom proximalen Ende des Abschnitts 43
- s₂: axiale Länge des Abschnitts 43

- d₁: Dicke der Haut
- s₁: Abstand der Nadelspitze der Vorstichhilfe zur Haut
- d₂: Abstand des Anschlags vom proximalen Ende des Abschnitts 43
- s₂: axiale Länge des Abschnitts 43

## Patentansprüche

1. Lichtapplikatorsystem (19) zur Untersuchung und/oder Behandlung von einem organischen Körper (9), wobei das Lichtapplikatorsystem (19) zumindest einen Lichtapplikator (1) und ein Positionierungselement (5) aufweist,
wobei der Lichtapplikator (1) einen distalseitigen Einführabschnitt (13) mit zumindest einem aktiv lichtemittierenden Element und einer in Bezug auf den Einführabschnitt (13) axial zentrisch angeordneten Nadelspitze (25) am distalen Ende zum Einstechen in Gewebe (11) des organischen Körpers (9) aufweist,
wobei das Positionierungselement (5) zumindest temporär in einer definierten Position und Ausrichtung relativ zum organischen Körper (9) fixierbar ist und zumindest eine Aufnahme (3) für den mindestens einen Lichtapplikator (1) aufweist, in welcher der mindestens eine Lichtapplikator (1) zumindest temporär eine definierte Position und Ausrichtung zum organischen Körper (9) aufweist und in welcher die Nadelspitze (25) des Lichtapplikators (1) in Bezug auf die zumindest eine Aufnahme (3) des Positionierungselements (5) axial zentriert geführt ist,
**dadurch gekennzeichnet, dass** das Lichtapplikatorsystem (19) eine Vorstichhilfe (21) mit einem Vorstichhilfeschaft (23) und einer in Bezug auf den Vorstichhilfeschaft (23) axial zentrisch angeordneten distalen Nadelspitze (15) aufweist, wobei die Vorstichhilfe (21) ebenfalls derart in die zumindest eine Aufnahme (3) des Positionierungselements (5) passt, dass die Nadelspitze (15) der Vorstichhilfe (21) in Bezug auf die zumindest eine Aufnahme (3) des Positionierungselements (5) axial zentriert in dieser Aufnahme (3) geführt wird, und dass die Vorstichhilfe (21) zumindest temporär eine definierte Ausrichtung zum organischen Körper (9) hat, die der definierten Ausrichtung zum organischen Körper (9) des mindestens einen Lichtapplikators (1) entspricht, wobei das Lichtapplikatorsystem (19) ferner einen Anschlag (35) aufweist, der eine maximale Vorstichtiefe der Vorstichhilfe (21) definiert, wobei die Vorstichhilfe (21) in den Lichtapplikator (1) integriert ist, wobei die Nadelspitze (15) der Vorstichhilfe (21) eine sich zumindest teilweise distal von dem aktiv lichtemittierenden Element angeordnete und sich distalwärts spitz verjüngende Nadelspitze (25) des Lichtapplikators (1) mit einem lichttransparenten Streukörper zur Streuung des Lichts des aktiv lichtemittierenden Elements ist, wobei der Anschlag (35) derart positionierbar und/oder formbar ist, dass er in einer Anschlagsposition eine maximale Vorstichtiefe des Lichtapplikators (1) definiert und in einer Nichtanschlagsposition eine größere Einstichtiefe des Lichtapplikators (1) erlaubt.

2. Lichtapplikatorsystem (19) nach Anspruch 1, wobei die Vorstichhilfe (21) eine vom Lichtapplikator (1) separate Komponente ist, welche nach einem Vorstich in den organischen Körper (9) aus der zumindest einen Aufnahme (3) des Positionierungselements (5) entnehmbar ist, um anschließend den Lichtapplikator (1) zur Untersuchung und/oder Behandlung des organischen Körpers (9) durch die zumindest eine Aufnahme (3) des Positionierungselements (5) in den vorgestochenen organischen Körper (9) einzuführen.

3. Lichtapplikatorsystem (19) nach einem der vorhergehenden Ansprüche, wobei die Nadelspitze (25) des Lichtapplikators (1) in Bezug auf die zumindest eine Aufnahme (3) des Positionierungselements (5) dadurch axial zentriert in der zumindest einen Aufnahme (3) geführt ist, dass ein Außendurchmesser des Einführabschnitts (13) des Lichtapplikators (1) oder ein Außendurchmesser einer auf den Außendurchmesser des Einführabschnitts (13) passenden Adapterhülse (31) an einen Innendurchmesser der Aufnahme (3) angepasst ist, und
wobei die Nadelspitze (15) der Vorstichhilfe (21) in Bezug auf die zumindest eine Aufnahme (3) dadurch axial zentriert in der Aufnahme (3) geführt ist, dass ein Außendurchmesser des Vorstichhilfeschafts (23) oder ein Außendurchmesser einer auf den Außendurchmesser des Vorstichhilfeschafts (23) passenden Adapterhülse (31) an den Innendurchmesser der Aufnahme (3) angepasst ist.

4. Lichtapplikatorsystem (19) nach einem der vorhergehenden Ansprüche, wobei der Einführabschnitt (13) des Lichtapplikators (1) eine sich zumindest teilweise distal von dem aktiv lichtemittierenden Element angeordnete und sich distalwärts spitz verjüngende Nadelspitze (25) mit einem lichttransparenten Streukörper zur Streuung des Lichts des aktiv lichtemittierenden Elements aufweist.

5. Lichtapplikatorsystem (19) nach einem der vorhergehenden Ansprüche, wobei die Nadelspitze (15) der Vorstichhilfe (21) eine oder mehr Schneidkanten (29) aufweist.

6. Lichtapplikatorsystem (19) nach Anspruch 5, wobei zumindest eine der Schneidkanten (29) durch eine distal vorstehende Schneidklinge (28) ausgebildet ist.

7. Lichtapplikatorsystem (19) nach einem der vorhergehenden Ansprüche, wobei die Vorstichhilfe (21) einen Vorstichhilfeschaft (23) mit einer axialen Länge L1 und einem Durchmesser D1 aufweist, die ein erstes Länge-Durchmesser-Verhältnis L1/D1 bestimmen, und wobei der Einführabschnitt (13) des Lichtapplikators (1) eine axiale Länge L2 und einen Durchmesser D2 aufweist, die ein zweites Länge-Durchmesser-Verhältnis L2/D2 bestimmen, wobei das zweite Länge-Durchmesser-Verhältnis L1/D1 wesentlich größer ist als das erste Länge-Durchmesser-Verhältnis L2/D2.

8. Lichtapplikatorsystem (19) nach einem der vorhergehenden Ansprüche, wobei die Vorstichhilfe (21) einen Vorstichhilfeschaft (23) mit einem Durchmesser (D1) aufweist, der passgenau in einen Innendurchmesser der mindestens einen Aufnahme des Positionierungselements passt und größer als ein Durchmesser (D2) des Einführabschnitts (13) des Lichtapplikators (1) ist, wobei das Lichtapplikatorsystem (19) ferner eine Adapterhülse (31) aufweist, deren Innendurchmesser passgenau dem Durchmesser (D2) des Einführabschnitts (13) des Lichtapplikators (1) entspricht und die zumindest abschnittsweise einen Außendurchmesser aufweist, der passgenau in einen Innendurchmesser der mindestens einen Aufnahme (3) des Positionierungselements (5) passt.

9. Lichtapplikatorsystem (19) nach einem der vorhergehenden Ansprüche, wobei die Adapterhülse (31) als Schutzhülse einer Nadelspitze des Einführabschnitts (13) des Lichtapplikators (1) fungiert und mit dem Einführabschnitt (13) axial beweglich und unverlierbar gekoppelt ist.

10. Lichtapplikatorsystem (19) nach einem der vorhergehenden Ansprüche, wobei die Vorstichhilfe (21) eine Stabilisationshülse (33) aufweist, die axial auf einem Vorstichhilfeschaft (23) der Vorstichhilfe (21) beweglich und in einer wählbaren Axialposition auf dem Vorstichhilfeschaft (23) fixierbar ist.

11. Lichtapplikatorsystem (19) nach einem der vorhergehenden Ansprüche, wobei die Vorstichhilfe (21) und/oder der Lichtapplikator (1) proximalseitig ein Griffelement (17, 27) zur manuellen Positionierung und Ausrichtung aufweist.

12. Lichtapplikatorsystem (19) nach Anspruch 11, wobei die Axialposition der Vorstichhilfe (21) und/oder des Einführabschnitts (13) relativ zum Positionierungselement (5) manuell durch Positionieren des Griffelements (17, 27) in Axialrichtung einstellbar ist.

13. Lichtapplikatorsystem (19) nach Anspruch 11 oder 12, wobei das Griffelement (17, 27) den Anschlag (35) bildet und axial auf der Vorstichhilfe (21) und/oder dem Einführabschnitt (13) beweglich und in einer wählbaren Axialposition fixierbar ist.

14. Lichtapplikatorsystem (19) nach einem der vorhergehenden Ansprüche, ferner mit einer Widerstandskomponente (37), welche eine proximalwärtige Widerstandskraft gegen eine distalwärtig manuell auf die Vorstichhilfe (21) ausgeübte Kraft bis zu einer maximalen Widerstandskraft ausübt und die Vorstichhilfe (21) schlagartig distalwärts freigibt, sobald die distalwärtige manuelle Kraft auf die Vorstichhilfe (21) die maximale Widerstandskraft überschreitet.

15. Lichtapplikatorsystem (19) nach Anspruch 14, wobei die Widerstandskomponente (37) zumindest teilweise elastisch federnd verformbar und durch die distalwärtige manuelle Kraft spannbar ist.

16. Lichtapplikatorsystem (19) nach Anspruch 14 oder 15, wobei die Widerstandskomponente (37) mindestens ein sich im Wesentlichen axial erstreckendes Federbein (39) mit Fuß (41) aufweist, wobei der Fuß (41) unter Spannung des Federbeins (39) die distalwärtige manuelle Kraft radial weggedrückt wird und bei Überschreiten der maximalen Widerstandskraft unter schlagartiger Entspannung des Federbeins (39) zurückschnappt.

## Claims

1. A light applicator system (19) for examining and/or treating an organic body (9), wherein the light applicator system (19) has at least one light applicator (1) and a positioning element (5),
wherein the light applicator (1) has a distal-side insertion portion (13) comprising at least one element that actively emits light and a needle tip (25), which is arranged centrally in the axial direction with respect to the insertion portion (13), on the distal end in order to pierce into tissue (11) of the organic body (9),
wherein the positioning element (5) is at least temporarily fixable in a defined position and orientation relative to the organic body (9) and has at least one receptacle (3) for the at least one light applicator (1), in which the at least one light applicator (1) at least temporarily has a defined position and orientation relative to the organic body (9) and in which the needle tip (25) of the light applicator (1) is guided in an axially centred manner with respect to the at least one receptacle (3) of the positioning element (5),
**characterised in that** the light applicator system (19) has a pre-piercing aid (21) comprising a pre-piercing aid shaft (23) and a distal needle tip (15) which is arranged in an axially centred manner with respect to the pre-piercing aid shaft (23), wherein the pre-piercing aid (21) likewise fits into the at least one receptacle (3) of the positioning element (5) such that the needle tip (15) of the pre-piercing aid (21) is guided in an axially centred manner in this receptacle (3) with respect to the at least one receptacle (3) of the positioning element (5), and **in that** the pre-piercing aid (21) at least temporarily has a defined orientation relative to the organic body (9), said orientation corresponding to the defined orientation of the at least one light applicator (1) relative to the organic body (9), wherein the light applicator system (19) further has a stop (35), which defines a maximum pre-piercing depth of the pre-piercing aid (21), wherein the pre-piercing aid (21) is integrated into the light applicator (1), wherein the needle tip (15) of the pre-piercing aid (21) is a needle tip (25) of the light applicator (1) that is arranged at least partially distally from the actively light-emitting element and tapers to a point distally and has a light-transparent scatter body for scattering the light of the actively light-emitting element, wherein the stop (35) is positionable and/or formable in such a way that, in a stop position, it defines a maximum pre-piercing depth of the light applicator (1) and, in a non-stop position, it allows a greater piercing depth of the light applicator (1).

2. The light applicator (19) according to claim 1, wherein the pre-piercing aid (21) is a component which is separate from the light applicator (1) and which, following a pre-piercing made in the organic body (9), is removable from the at least one receptacle (3) of the positioning element (5) in order to then introduce the light applicator (1), for examination and/or treatment of the organic body (9), through the at least one receptacle (3) of the positioning element (5) into the pre-pierced organic body (9).

3. The light applicator system (19) according to any one of the preceding claims, wherein the needle tip (25) of the light applicator (1) is guided in the at least one receptacle (3) in an axially centred manner with respect to the at least one receptacle (3) of the positioning element (5) in that an outer diameter of the insertion portion (13) of the light applicator (1) or an outer diameter of an adapter sleeve (31) matching the outer diameter of the insertion portion (13) is matched to an inner diameter of the receptacle (3), and
wherein the needle tip (15) of the pre-piercing aid (21) is guided in the receptacle (3) in an axially centred manner with respect to the at least one receptacle (3) in that an outer diameter of the pre-piercing aid shaft (23) or an outer diameter of an adapter sleeve (31) matching the outer diameter of the pre-piercing shaft (23) is matched to the inner diameter of the receptacle (3).

4. The light applicator system (19) according to any one of the preceding claims, wherein the insertion portion (13) of the light applicator (1) has a needle tip (25) that is arranged at least partially distally from the actively light-emitting element and tapers to a point distally and has a light-transparent scatter body for scattering the light of the actively light-emitting element.

5. The light applicator system (19) according to any one of the preceding claims, wherein the needle tip (15) of the pre-piercing aid (21) has one or more cutting edges (29).

6. The light applicator system (19) according to claim 5, wherein at least one of the cutting edges (29) is formed by a distally protruding cutting edge (28).

7. The light applicator system (19) according to any one of the preceding claims, wherein the pre-piercing aid (21) has a pre-piercing aid shaft (23) with an axial length L1 and a diameter D1, which determine a first length to diameter ratio L1/D1, and wherein the insertion portion (13) of the light applicator (1) has an axial length L2 and a diameter D2, which determine a second length to diameter ratio L2/D2, wherein the second length to diameter ration L1/D1 is significantly greater than the first length to diameter ratio L2/D2.

8. The light applicator system (19) according to any one of the preceding claims, wherein the pre-piercing aid (21) has a pre-piercing aid shaft (23) with a diameter (D1), which fits accurately into an inner diameter of the at least one receptacle of the positioning element and is larger than a diameter (D2) of the insertion portion (13) of the light applicator (1), wherein the light applicator system (19) further has an adapter sleeve (31), the inner diameter of which corresponds accurately to the diameter (D2) of the insertion portion (13) of the light applicator (1) and which has, at least in portions, an outer diameter that fits accurately in an inner diameter of the at least one receptacle (3) of the positioning element (5).

9. The light applicator system (19) according to any one of the preceding claims, wherein the adapter sleeve (31) functions as a protective sleeve of a needle tip of the insertion portion (13) of the light applicator (1) and is coupled in an axially movable manner to the insertion portion (13) in such a way that the adapter sleeve cannot be lost.

10. The light applicator system (19) according to any one of the preceding claims, wherein the pre-piercing aid (21) has a stabilisation sleeve (33), which is axially movable on a pre-piercing aid shaft (23) of the pre-piercing aid (21) and fixable in a selectable axial position on the pre-piercing aid shaft (23).

11. The light applicator system (19) according to any one of the preceding claims, wherein the pre-piercing aid (21) and/or the light applicator (1) has a grip element (17, 27) proximally for manual positioning and orientation.

12. The light applicator system (19) according to claim 11, wherein the axial position of the pre-piercing aid (21) and/or the insertion portion (13) relative to the positioning element (5) is adjustable manually by positioning of the grip element (17, 27) in the axial direction.

13. The light applicator system (19) according to claim 11 or 12, wherein the grip element (17, 27) forms the stop (35) and is movable axially on the pre-piercing aid (21) and/or the insertion portion (13) and is fixable in a selectable axial position.

14. The light applicator system (19) according to any one of the preceding claims, further comprising a resistance component (37), which exerts a proximally directed resistance force against a distally directed force exerted manually onto the pre-piercing aid (21) up to a maximum resistance force and suddenly releases the pre-piercing aid (21) distally as soon as the distally directed manual force on the pre-piercing aid (21) exceeds the maximum resistance force.

15. The light applicator system (19) according to claim 14, wherein the resistance component (37) is elastically resiliently deformable at least in part and can be tensioned by the distally directed manual force.

16. The light applicator system (19) according to claim 14 or 15, wherein the resistance component (37) has at least one substantially axially extending spring leg (39) with foot (41), wherein the foot (41) is pushed away radially by the distally directed manual force with tensioning of the spring leg (39) and snaps back with sudden relief of the spring leg (39) when the maximum resistance force is exceeded.

## Revendications

1. Système d'applicateur de lumière (19) pour examiner et/ou traiter un corps organique (9), dans lequel le système d'applicateur de lumière (19) présente au moins un applicateur de lumière (1) et un élément de positionnement (5),
dans lequel l'applicateur de lumière (1) présente un tronçon d'introduction (13) côté distal avec au moins un élément actif émetteur de lumière et une pointe d'aiguille (25) à l'extrémité distale, disposée en étant centrée axialement par rapport au tronçon d'introduction (13) pour piquer dans le tissu (11) du corps organique (9),
dans lequel l'élément de positionnement (5) peut être fixé au moins temporairement dans une position et un alignement définis par rapport au corps organique (9) et présente au moins un logement (3) pour l'au moins un applicateur de lumière (1), dans lequel l'au moins un applicateur de lumière (1) présente au moins temporairement une position et un alignement définis par rapport au corps organique (9) et dans lequel la pointe d'aiguille (25) de l'applicateur de lumière (1) est dirigée en étant centrée axialement par rapport à l'au moins un logement (3) de l'élément de positionnement (5),
**caractérisé en ce que** le système d'applicateur de lumière (19) présente un auxiliaire de pré-piquage (21) avec une tige d'auxiliaire de pré-piquage (23) et une pointe d'aiguille (15) distale disposée en étant centrée axialement par rapport à la tige d'auxiliaire de pré-piquage (23), dans lequel l'auxiliaire de pré-piquage (21) va également de telle façon dans l'au moins un logement (3) de l'élément de positionnement (5) que la pointe d'aiguille (15) de l'auxiliaire de pré-piquage (21), par rapport à l'au moins un logement (3) de l'élément de positionnement (5), est dirigée en étant centrée axialement dans ce logement (3), et que l'auxiliaire de pré-piquage (21) a un alignement défini au moins temporairement par rapport au corps organique (9) qui correspond à l'alignement défini de l'au moins un applicateur de lumière (1) par rapport au corps organique (9), dans lequel le système d'applicateur de lumière (19) présente en outre une butée (35) qui définit une profondeur de pré-piquage maximale de l'auxiliaire de pré-piquage (21), dans lequel l'auxiliaire de pré-piquage (21) est intégré dans l'applicateur de lumière (1), dans lequel la pointe d'aiguille (15) de l'auxiliaire de pré-piquage (21) est une pointe d'aiguille (25) de l'applicateur de lumière (1) disposée au moins partiellement de manière distale par rapport à l'élément actif émetteur de lumière et se réduisant en pointe dans le sens distal avec un corps diffuseur transparent à la lumière pour diffuser la lumière de l'élément actif émetteur de lumière, dans lequel la butée (35) peut être ainsi positionnée et/ou formée qu'elle définit une profondeur de pré-piquage maximale de l'applicateur de lumière (1) dans une position de butée et permet une plus grande profondeur de piquage de l'applicateur de lumière (1) dans une position en non-butée.

2. Système d'applicateur de lumière (19) selon la revendication 1, dans lequel l'auxiliaire de pré-piquage (21) est une composante séparée de l'applicateur de lumière (1), laquelle peut être enlevée de l'au moins un logement (3) de l'élément de positionnement (5) après un pré-piquage dans le corps organique (9) pour introduire ensuite l'applicateur de lumière (1) à travers l'au moins un logement (3) de l'élément de positionnement (5) dans le corps (9) organique pré-piqué pour l'examen et/ou le traitement du corps organique (9).

3. Système d'applicateur de lumière (19) selon l'une des revendications précédentes, dans lequel la pointe d'aiguille (25) de l'applicateur de lumière (1), par rapport à l'au moins un logement (3) de l'élément de positionnement (5), est dirigée en étant centrée axialement dans l'au moins un logement (3) de telle façon qu'un diamètre extérieur du tronçon d'introduction (13) de l'applicateur de lumière (1) ou un diamètre extérieur d'une douille adaptatrice (31) adaptée au diamètre extérieur du tronçon d'introduction (13) est adapté à un diamètre intérieur du logement (3), et
dans lequel la pointe d'aiguille (15) de l'auxiliaire de pré-piquage (21) est dirigée dans le logement (3) en étant centrée axialement par rapport à l'au moins un logement (3) de telle façon qu'un diamètre extérieur de la tige d'auxiliaire de pré-piquage (23) ou un diamètre extérieur d'une douille adaptatrice (31) adaptée au diamètre extérieur de la tige d'auxiliaire de pré-piquage (23) est adapté au diamètre intérieur du logement (3).

4. Système d'applicateur de lumière (19) selon l'une des revendications précédentes, dans lequel le tronçon d'introduction (13) de l'applicateur de lumière (1) présente une pointe d'aiguille (25) disposée au moins partiellement de manière distale par rapport à l'élément actif émetteur de lumière et se réduisant en pointe dans le sens distal avec un corps diffuseur transparent à la lumière pour diffuser la lumière de l'élément actif émetteur de lumière.

5. Système d'applicateur de lumière (19) selon l'une des revendications précédentes, dans lequel la pointe d'aiguille (15) de l'auxiliaire de pré-piquage (21) présente une ou plusieurs arête(s) de coupe (29).

6. Système d'applicateur de lumière (19) selon la revendication 5, dans lequel au moins une des arêtes de coupe (29) est formée par une lame de coupe (28) en saillie de manière distale.

7. Système d'applicateur de lumière (19) selon l'une des revendications précédentes, dans lequel l'auxiliaire de pré-piquage (21) présente une tige d'auxiliaire de pré-piquage (23) avec une longueur axiale L1 et un diamètre D1 qui déterminent un premier rapport longueur-diamètre L1/D1, et dans lequel le tronçon d'introduction (13) de l'applicateur de lumière (1) présente une longueur axiale L2 et un diamètre D2 qui déterminent un second rapport longueur-diamètre L2/D2, dans lequel le deuxième rapport longueur-diamètre L1/D1 est essentiellement plus grand que le premier rapport longueur-diamètre L2/D2.

8. Système d'applicateur de lumière (19) selon l'une des revendications précédentes, dans lequel l'auxiliaire de pré-piquage (21) présente une tige d'auxiliaire de pré-piquage (23) avec un diamètre (D1) qui va précisément dans un diamètre intérieur de l'au moins un logement de l'élément de positionnement et est plus grand qu'un diamètre (D2) du tronçon d'introduction (13) de l'applicateur de lumière (1), dans lequel le système d'applicateur de lumière (19) présente en outre une douille adaptatrice (31) dont le diamètre intérieur correspond précisément au diamètre (D2) du tronçon d'introduction (13) de l'applicateur de lumière (1) et qui présente au moins par tronçon un diamètre extérieur qui va précisément dans un diamètre intérieur de l'au moins un logement (3) de l'élément de positionnement (5).

9. Système d'applicateur de lumière (19) selon l'une des revendications précédentes, dans lequel la douille adaptatrice (31) sert de douille protectrice d'une pointe d'aiguille du tronçon d'introduction (13) de l'applicateur de lumière (1) et est couplée avec le tronçon d'introduction (13) en étant imperdable et mobile axialement.

10. Système d'applicateur de lumière (19) selon l'une des revendications précédentes, dans lequel l'auxiliaire de pré-piquage (21) présente une douille stabilisatrice (33) qui est mobile axialement sur une tige d'auxiliaire de pré-piquage (23) de l'auxiliaire de pré-piquage (21) et peut être fixée dans une position axiale au choix sur la tige d'auxiliaire de pré-piquage (23).

11. Système d'applicateur de lumière (19) selon l'une des revendications précédentes, dans lequel l'auxiliaire de pré-piquage (21) et/ou l'applicateur de lumière (1) présente(nt) côté proximal un élément de poignée (17, 27) pour le positionnement et l'alignement manuels.

12. Système d'applicateur de lumière (19) selon la revendication 11, dans lequel la position axiale de l'auxiliaire de pré-piquage (21) et/ou du tronçon d'introduction (13) peut être réglée manuellement par rapport à l'élément de positionnement (5) en positionnant l'élément de poignée (17, 27) dans le sens axial.

13. Système d'applicateur de lumière (19) selon la revendication 11 ou 12, dans lequel l'élément de poignée (17, 27) forme la butée (35) et est mobile axialement sur l'auxiliaire de pré-piquage (21) et/ou le tronçon d'introduction (13) et peut être fixé dans une position axiale au choix.

14. Système d'applicateur de lumière (19) selon l'une des revendications précédentes, comprenant en outre une composante de résistance (37), laquelle exerce une force de résistance dans le sens proximal contre une force exercée manuellement dans le sens distal sur l'auxiliaire de pré-piquage (21) jusqu'à une force de résistance maximale et libère l'auxiliaire de pré-piquage (21) de manière soudaine dans le sens distal dès que la force manuelle dans le sens distal sur l'auxiliaire de pré-piquage (21) dépasse la force de résistance maximale.

15. Système d'applicateur de lumière (19) selon la revendication 14, dans lequel la composante de résistance (37) peut être déformée au moins partiellement de manière élastique et peut être tendue par la force manuelle dans le sens distal.

16. Système d'applicateur de lumière (19) selon la revendication 14 ou 15, dans lequel la composante de résistance (37) présente une jambe de force (39) avec pied (41) s'étendant essentiellement axialement, dans lequel le pied (41), par une tension de la jambe de force (39), est éloigné radialement par la force manuelle dans le sens distal et se rabat par une détente soudaine de la jambe de force (39) lorsque la force de résistance maximale est dépassée.
